# EUROPEAN PATENT SPECIFICATION

(11) **EP 1 117 677 B1**
(45) Date of publication and mention of the grant of the patent: **12.11.2003**
(21) Application number: 99947256.6
(22) Date of filing: 29.09.1999
(51) Int. Cl.: C07K 1/04, C07K 7/02, C07K 14/00, A61K 39/04, A61K 39/02, A61K 39/385

(54) **LIGAND PRESENTING ASSEMBLY (LPA), METHOD OF PREPARATION AND USES THEREOF**
"LIGAND PRESENTING ASSEMBLY" (LPA), VERFAHREN ZU DESSEN HERSTELLUNG UND VERWENDUNGEN
ENSEMBLE DE PRESENTATION DE LIGANDS (LPA) SON PROCEDE DE PREPARATION ET SES UTILISATIONS

(30) Priority: 29.09.1998 DK 123398
(43) Date of publication of application: 25.07.2001
(73) Proprietor: Statens Serum Institut, 2300 Copenhagen S (DK); Holm, Arne, 2942 Skodsborg (DK)
(72) Inventor: HOLM, Arne, DK-2942 Skodsborg (DK); JORGENSEN, Rikke, Malene, DK-2800 Lyngby (DK); OSTERGAARD, Soren, DK-2700 Bronshoj (DK); THEISEN, Michael, 1957 Frederiksberg C (DK)
(74) Representative: Olsen, Lars Pallisgaard
(86) International application number: DK9900510
(87) International publication number: WO00018791

(56) References cited:
- WO-A-94/02506
- WO-A-97/42221
- WO-A-98/32469
- TAM J P: "Recent advances in multiple antigen peptides" JOURNAL OF IMMUNOLOGICAL METHODS, vol. 196, no. 1, 1 January 1996 (1996-01-01), page 17-32 XP004021277 cited in the application
- PELUSO S ET AL: "Protein Mimetics (TASP) by Sequential Condensation of Peptide Loops to an Immobilised Topological Template" TETRAHEDRON, vol. 53, no. 21, 26 May 1997 (1997-05-26), page 7231-7236 XP004105702
- G. TUCHSCHERER ET AL: "Templates in protein de novo design" J. BIOTECHNOLOGY, vol. 41, 1995, pages 197-210, XP002104629
- M. HARBOE ET AL.: "B-cell epitopes and Quantification of ESAT-6 protein of Mycobacterium tuberculosis" INFECTION AND IMMUNITY, vol. 66, no. 2, February 1998 (1998-02), pages 717-723, XP002104630

## Description

The present invention relates to a method for preparing a ligand presenting assembly (LPA), an LPA, an immunological composition and a vaccine. The invention further relates to a method for generating antibodies, a kit for use in diagnosis and use of an LPA for preparing a pharmaceutical composition.

### BACKGROUND OF THE INVENTION

Peptides having an amino acid sequence corresponding to a part of the amino acid sequence of a protein or antigen are often used in immunoassays for screening for antibodies or preparation of vaccines.

The peptides or immunogenic compounds are often coupled to various carriers in order to enhance the immunogenic effect. One such system is multiple presentation of antigenic peptides, which has long been recognised as valuable means for amplifying peptide immunogens. Most commonly an antigen is attached to a natural carrier such as a protein, a carbohydrate, a lipid or a liposome to provide a vaccine component or a synthetic sero-diagnostic.

There are, however, a number of problems with these products which may be related to the carriers. Since these are isolated from natural sources they may not be of uniform quality and may further contain harmful contaminants being themselves antigenic. When used for vaccines they may in particular result in fevers and tissue swelling. In attempts to overcome certain of these problems much research has in later years been focused on antigen presentation using purely synthetic carriers.

Dendritic polymers are a such class of polymers or synthetic proteins. Such polymers have been described widely in the literature. The polymers are often referred to as dendritic because of the similarity with a branched tree.

A multimerisation known as multiple antigen peptide (MAP) using a branched architecture with a controlled and limited array of branches was described by Tam in PCT/-US90/02039 (ref. 1).

MAPs have applications as synthetic vaccines and as diagnostic tools as well as other biochemical uses including immunoassays, serodiagnosis, epitope mapping, inhibitors, artificial proteins, intracellular delivery, affinity purification of antibodies and presentation of T-cell epitopes (ref. 2).

All branched peptides are similar in that they branch from a core matrix or template which provide them with different dendrimeric properties (ref. 3, ref. 4). Most commonly, lysine is used as amino acid in the core matrix because it has two ends, the α- and ε-amino groups, available for branching reactions.

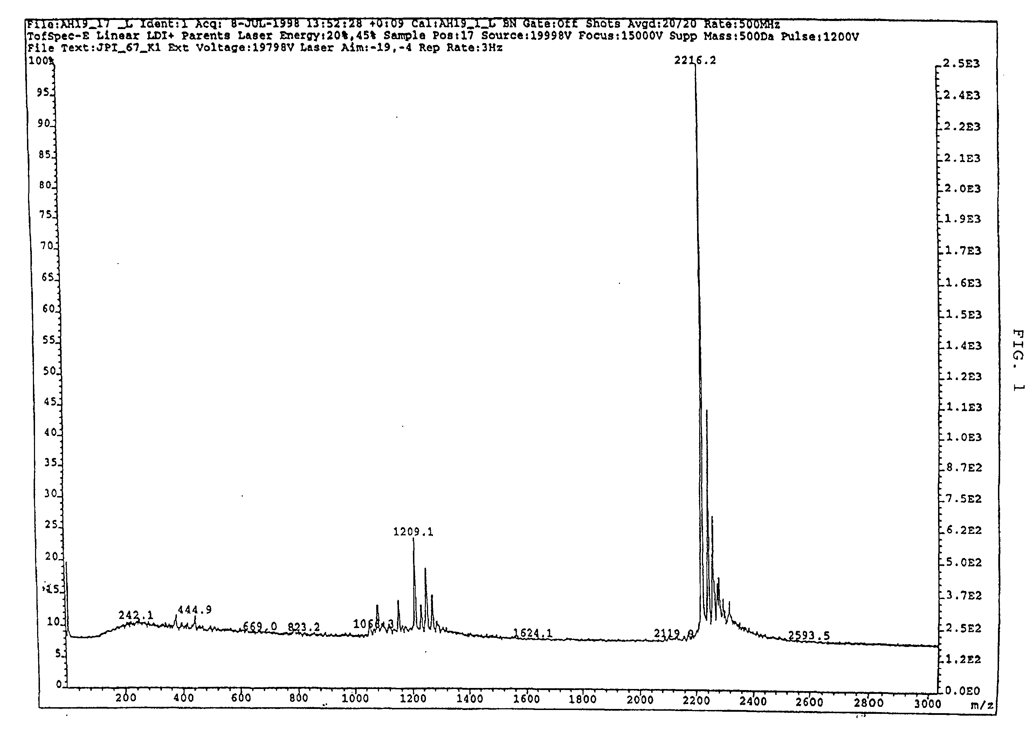

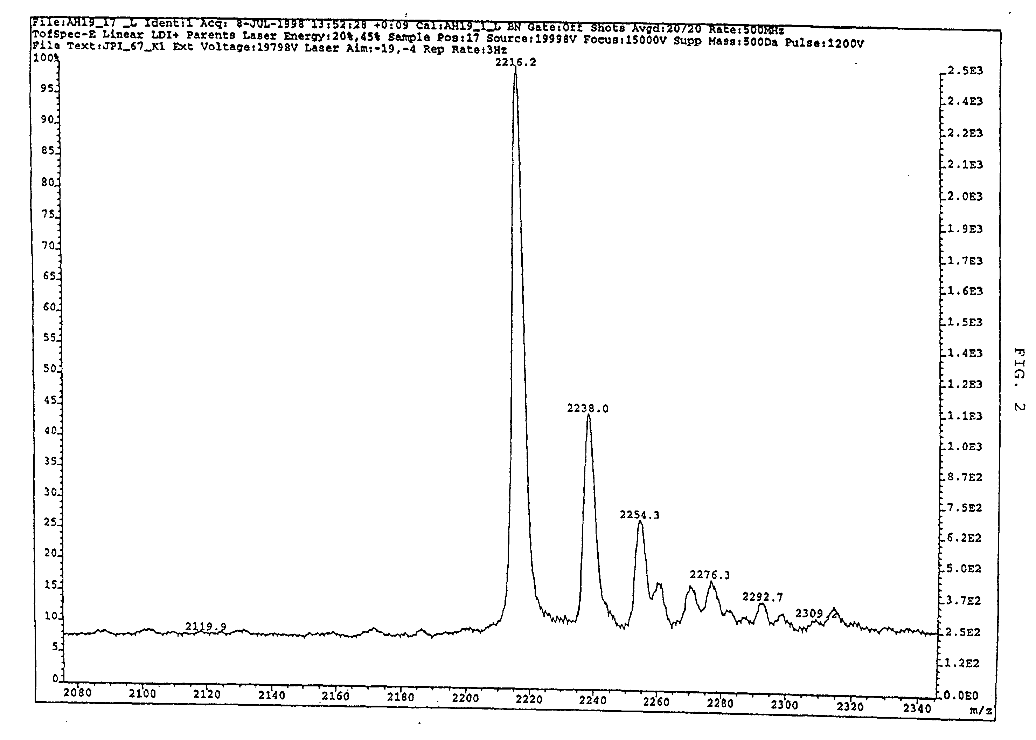

Different types of branching have been reviewed recently (ref. 5, ref. 2) including cascade, pennant or radial type of branching arrangement (Fig. 1A, B and C in ref. 5). A stem peptide carrier has been reported which is a short lipopeptide carrier, the stem peptide, which is useful for side-chain presentation of peptides (Fig. 2) (ref. 6).

The MAPS described above all have in common that an amino group is the starting point for the antigen synthesis resulting in peptides which are attached to the core matrix by the C-terminal end and, thus, having a free N-terminal end.

The effectiveness of a B cell epitope on MAP to elicit humoral responses depends on the presence of a T cell recognition site located in the stretch of amino acids. While this is not always the case, chimeric B-T constructs have been made producing strongly enhanced immune responses. Such MAPS are known as diepitope MAPs. The effectiveness of diepitope MAPs over monoepitope MAP has among other been studied in synthetic vaccine models of the human immunodeficiency virus (HIV) type 1. There are many possible ways of arranging B and T cell epitopes on MAPs. Most commonly, they are linked in a tandem arrangement. High antibody titers were elicited by MAPs containing equimolar amounts of the T and B epitopes, while monoepitope MAPs and B cell epitope monomers showed no immunogenic effect in A/J mice used in the experiments, cf. ref. 5. MAPs may be lipidated by standard peptide synthesis to produce a full range of response without any extraneous adjuvant (ref. 8, ref. 9).

As mentioned above, MAPs have a number of other applications. Important areas are immunoassays and serodiagnosis of e.g. malaria, cirrhosis, HIV-1, schistoma mansoni, systemic lupus erythematosus, and Epstein-Barr virus (EBV).

Most studies of MAPs have been carried out using tetra- or octameric MAP. However, it is generally found that with peptides composed of more than 15 amino acids, no real advantage is obtained using an octameric as compared to a tetrameric MAP (ref. 2).

In general, two methods can be used for MAP synthesis, namely the direct route and the indirect route. In both cases, the C-terminal core is first assembled on a solid support using diprotected Boc-Lys(Boc) in the case of Boc chemistry, or Fmoc-Lys(Fmoc) in the case of Fmoc chemistry in order to obtain the desired degree of branching.

In the direct route, synthesis is continued by stepwise assembly of the particular peptide on the lysinyl core by the well-known Merrifield method. This stepwise method produces peptide antigens with a C to N orientation. B and T cell epitopes may be synthesised in tandem, or, alternatively, each epitope can be synthesised on the different lysine arms of the core using orthogonal deprotection methods. Such methods include i.a. Boc-Fmoc, and Fmoc-Aloc lysines. Although this approach for MAP synthesis is convenient, the quality of the final product may be questionable and problems of obtaining a well defined product have been discussed (ref. 10). Problems arise during chain assembly because MAPs are macromolecules where each single chain may interact with each other, preventing high efficiency couplings and leading to deletion sequences which require effort to purify. Furthermore, the characterisation of these products by, e.g. electrospray mass spectroscopy (ES-MS) is also difficult and as a result, multi-dendritic peptide immunogens have often been used without full characterisation. In the recent investigations by Keah et al. (ref. 10), a capping procedure after each amino acid coupling has been introduced in MAP synthesis, which improves the yield of the desired multi-dendritic immunogens. For analysis, an enzymatic (tryptic) digestion method with the MAP construct, followed by characterisation of the enzymatic digest by reversed phase high-performance liquid chromatography-ES-MS method has been developed (ref. 10).

For many immunisations with MAP, the heterogeneity is not necessarily critical but for clinical applications, it is absolutely necessary to have chemically unambiguous MAPS without side products.

These considerations led to the development of indirect methods for MAP synthesis, in which methods purified unprotected peptide segments are coupled to the core matrix. Two general methods for this type of ligation have been developed which, respectively, are based on thiol and carbonyl chemistries.

The thiol chemistry may be carried out by incorporation of chloroacetyl group(s) on the lysine matrix and subsequent coupling to a purified, synthetic N-terminal cysteinyl peptide to yield a MAP with unambiguous structure (ref. 11, ref. 12). The reverse placement may be achieved with thiol on the core matrix by using an S-acetyl group on the lysinyl core and the haloacetyl group on the peptide (ref. 13). The use of disulphide bond formation for MAP synthesis is an alternative which is a well-known process in protein and peptide chemistry (ref. 2). A drawback in this type of chemistry is among others interference with cysteine present in the native antigen.

In the carbonyl chemistry, condensation takes place between a carbonyl group and a weak base. There are two types of weak bases which may be used for this reaction. The first includes hydroxylamines and hydrazines, and the second includes 1,2-disubstituted moieties such as 1,2-aminoethanthiol and 1,2-aminoethanol. The two last groups are found in N-terminal cysteine and threonine, respectively, and the condensation products are thiazolidine and oxazolidine. The carbonyl group on the core matrix may be obtained by periodate oxidation of N-terminal serine, threonine or cysteine.

Several investigations along these approaches for MAP construction have been carried out to develop other methods for preparing peptide dendrimers providing flexibility for N or C attachment to the matrix core as reviewed by Tam (ref. 2). The processes may need optimised conditions and a major concern by the use of thiols in the thiazolidine formation is a side reaction due to oxidation to disulphides, leading to impure products.

In the direct MAP synthesis route with stepwise assembly of the desired peptide on the lysinyl core a peptide antigen having C to N orientation is obtained. Such a MAP may be useful for ELISA applications, where antigen recognition by an antibody is directed at the N-terminal or preferably close to the N-terminal, while recognition of the C-terminal may be hindered. This is e.g. the case with the dominant epitope of the Outer surface protein C (OspC) from Borrelia burgdorferi sensu lato causing Lyme borreliosis. Epitope mapping of OspC using sera from patients with neuroborreliosis led to identification of one single major immunodominant epitope within the C-terminal ten amino acid residues, the decapeptide PVVAESPKKP. Peptide binding studies and alanine scanning revealed a critical role for the PKKP-sequence and in particular its terminal carboxyl group for the binding of IgM antibodies from patients with Lyme borreliosis. Thus substitution of the C-terminal proline or replacement of the carboxy group with a carboxamido group greatly reduced the ability of the peptides to compete with OspC19-207 for the binding of IgM antibodies in all five sera investigated. This significant fact clearly exclude a MAP formed by stepwise synthesis on a lysine core as this results in C-terminal attachment to the core matrix.

From the above discussion, it is clear that a system providing a choice between C-, and C- and N-terminal presentation of sequences, and at the same time being easily performed and yielding improved products suitable for various applications, is needed.

### SUMMARY OF THE INVENTION

The present invention provides novel methods for preparing LPAs enabling presentation of desired sequence(s). In a further aspect, the present invention relates to a method for preparing LPAs enabling presentation of desired sequences(s) and chemical moieties.

In another aspect, the present invention relates to an LPA obtainable by the method.

In yet a further aspect, the present invention relates to immunological compositions and vaccines, as well as a method of generating antibodies in an animal, including a human being.

Furthermore, the present invention relates to the use of such LPAs in various applications and kits.

The present invention is described in detail below.

### BRIEF DESCRIPTION OF THE FIGURES

Figure 1 shows the MALDI-TOF MS spectrum of the crude product NH₂CH(CH₂CO-ProValValAlaGluSerProLysLysPro-OH)₂ (also denoted NH₂CH(CH₂CO-Seq. ID 1-OH)₂) [LPA-III] of Example 3.
Figure 2 shows the MALDI-TOF MS spectrum of the crude product NH₂CH(CH₂CO-ProValValAlaGluSerProLysLysPro-OH)₂ (also denoted NH₂CH(CH₂CO-Seq. ID 1-OH)₂) [LPA-III] of Example 3.
Figure 3 shows the HPLC chromatogram of crude H-ProValValAlaGluSerProLysLysPro-OH, cf. Example 3.
Figure 4 shows the HPLC chromatogram of crude NH₂CH-(CH₂CO-ProValValAlaGluSerProLysLysPro-OH)₂ (also denoted NH₂CH(CH₂CO-Seq. ID 1-OH)₂) [LPA-III] of Example 3.
Figure 5 shows the HPLC chromatogram of the HPLC purified product NH₂CH(CH₂CO-ProValValAlaGluSerProLysLysPro-OH)₂ (also denoted NH₂CH(CH₂CO-Seq. ID 1-OH)₂) [LPA-III] of Example 3.
Figure 6 shows the MALDI-TOF MS spectrum of the crude product H-Lys-NHCH(CH₂CO-ProValValAlaGluSerProLysLysPro-OH)₂ (also denoted H-Lys-NHCH(CH₂CO-Seq. ID 1-OH)₂) [LPA-IV] of Example 4.
Figure 7 shows the ELISA results from Example 13.
Figure 8 shows the ELISA results from Example 14.
Figure 9 shows the MALDI-TOF MS spectrum of the crude product CH₂(CH₂CO-β-Ala-β-AlaLysGluProAsnLysGlyValAsnPro-AspGluValβAla)₂ also denoted CH₂(CH₂CO-β-Ala-β-Ala-Seq. ID 4-β-Ala)₂ [LPA-VII] of Example 7.
Figure 10 shows the MALDI-TOF MS spectrum of the gel filtrated product HC(CH₂CO-LysGluProAsnLysGlyValAsnPro-AspGluValβAla)₂COOH also denoted HC(CH₂CO-Seq. ID 4-β-Ala)₂COOH [LPA-VIII] of Example 8.
Figure 11 shows the HPLC chromatogram of the gel filtrated product HC(CH₂CO-LysGluProAsnLysGlyValAsn-ProAspGluValβAla)₂COOH also denoted HC(CH₂CO-Seq. ID 4-βAla)₂COOH [LPA-VIII] of Example 8.
Figure 12 shows the MALDI-TOF MS spectrum of the crude product Fmoc-NHCH(CH₂CO-AspArgValTyrIleHisProPheHisLeu-NH₂)₂ also denoted Fmoc-NHCH(CH₂CO-Seq. ID 5-NH₂)₂ [LPA-IX] of Example 10.
Figure 13 shows the HPLC chromatogram of the crude product Fmoc-NHCH(CH₂CO-AspArgValTyrIleHisProPheHisLeu-NH₂)₂ also denoted Fmoc-NHCH(CH₂CO-Seq. ID 5-NH₂)₂ [LPA-IX] of Example 10.

### DETAILED DESCRIPTION OF THE INVENTION

In a first aspect, the present invention provides a novel method for preparing an LPA enabling presentation of desired sequence(s), which method comprises the steps of
(a) providing by solid phase synthesis or fragment coupling ligand(s) comprising the desired sequence(s), the ligand(s) being attached to a solid phase,
(b) if necessary, deprotecting any N-terminal amino groups while the ligand(s) are still attached to the solid phase,
(c) reacting the ligand(s) having unprotected N-terminal amino groups with an achiral di-, tri- or tetracarboxylic acid so as to provide a construct having a ring structure, and
(d) cleaving the construct from the solid phase so as to provide an LPA comprising ligand(s) having free C-terminal groups.

In a further aspect, the present invention provides a method for preparing an LPA enabling presentation of desired sequence(s), or desired sequence(s) and chemical moieties, which method comprises the steps of
(c¹) if present, prior to step (d), deprotecting any N-protected amino groups originating from the carboxylic acid used in step (c),
(c²) continuing the solid phase synthesis or fragment coupling so as to provide ligand(s) comprising desired sequence(s) having at least one N-protected N-terminal amino group and/or attaching chemical moieties, and
(c³) deprotecting, if present, any N-terminal amino group(s) prior to or after step (d).

A unique feature of the method according to the present invention is the step providing a ring structure. Surprisingly, by the method according to the present invention it is possible to prepare very long ring systems interconnected by reaction with the achiral di-, tri- or tetracarboxylic acid. The ring structure formed between desired sequences further enables additional presentation of desired sequence(s) and chemical moieties. Thus, the method of the invention is easily performed and, as mentioned above, step (c¹) and (c²) enable further presentation of desired sequences and/or chemical moieties. The desired sequences of step (c²) may be identical or different from the sequences of step (a). Thus, LPAs are suitable and very flexible systems for polyfunctional constructs, and furthermore, products of high purity are obtained.

Formation of double chain peptides having hemoregulatory activity is disclosed in WO 93/24523 (ref. 14). The two peptide chains are joined together at the Cα-atoms of non-terminal amino acids in equivalent positions in each peptide chain via a carbon-carbon bond or via divalent bridging group -A- where -A- is a C₁₋₆ carbon chain. The double chain peptides are constructed from a single chain of the general formula R^{a}-R^{b}-R^{c}-R^{d}-(R^{e})ₙ-R^{f} where R^{a}, R^{b}, R^{c}, and R^{d} represent selected amino acids and R^{e} is -NH-CH(R)-CO-, R^{f} represents lysine, arginine or glycine and n is 0 or 1. The bridging group A is attached to the Cα-atoms of amino acids R^{d} and bridging is accomplished by acylating the synthesis resin bearing the desired N-deprotected C-terminal residue with one half equivalent of Fmoc-protected diamino dicarboxylic acid of such a type as 2,7-diaminosuberic acid or 2,9-diaminosebacic acid with the aid of coupling agents DCC (dicyclohexyl carbodiimide) and HOBt. After deprotection peptide synthesis is continued with the remaining amino acids depending on the sequence in question. It is a characteristic and a necessary condition of the employed amino acids in order to obtain the claimed biologically active compounds that they contain two chiral centres. Furthermore, the peptide chains which are assembled with the said diamino dicarboxylic acid are limited to two amino acids each according to the depicted formula.

In recent investigations coupling with half equivalent of protected diamino dicarboxyl acids with synthesis resin bearing an N-deprotected C-terminal residue with up to three amino acids in the peptide chain has been reported (ref. 15, ref. 16). As to the conditions of the bridging technique it can be learned from the 1993 report by Alberts et al. (ref. 17) that bridging with half equivalent 2,7-bis(Boc-amino) suberic acid coupled to one equivalent of lysine attached to the synthesis resin (2Cl-Z) is slow and takes place over up to 4 days. Thus, ring formation of longer amino acid sequences may be similarly or more difficult requiring activation conditions which could prevent formation of well defined products with optically active bridging compounds.

The review by Tuchscherer and Mutter (ref. 18) deals with template-based de novo design of synthetic proteins (so-called TASPs, template-assembled synthetic proteins). De novo design of polypeptide sequences with a three-dimensional structure necessary for many biological functions is limited by the complex folding process of the polypeptide sequences. The problem of folding can be bypassed through constructing protein-like molecules with a built in device for intramolecular folding, namely proteins of non-natural chain architecture, i.e. TASPs. The concept is to use topological templates to introduce tertiary structures of the peptide sequences by directing attached peptide blocks to a predetermined three-dimensional packing arrangement. This method provides synthetic proteins in which a predetermined backbone confirmation act as host for selective attachment of functional sites (e.g. amino acid side chains or peptides) in spatially defined positions via a spacer group. An early example of this method is the use of β-D-glucose as a template (scaffold) for the design of a non-peptidic mimic of the somatostatin agonist SRIF. Covalent attachment of the amino acid side chains of Phe, Trp and Lys to D-glucose derivatives resulted in a molecule recognised by G-protein coupled receptors. The concept and purpose described in this reference is clearly different from the concept of the present invention. There is no mentioning of synthesis or assembly of ligands using achiral di-, tri- or tetracarboxylic acids.

In WO 94/02506 (ref. 19) MAP systems are disclosed. The MAPs are constructed with the following structure: Peptide antigen-core-linker-lipid anchor-resin. The core is based on lysine technology with four lysine arms onto which the peptide is assembled by conventional stepwise synthesis by reaction of a terminal functionality, e.g. a carboxylic acid group or an amine group. Different active substituents may be included in the molecule, thus providing MAPs with multiple antigenicities. In the document, the use of di-, tri- or tetracarboxylic acids is not mentioned or suggested. Thus, the present invention is clearly based on a different concept.

In WO 98/32469 (ref. 20) compounds comprising a linear, branched or dendrimeric polymer backbone are disclosed. At least one reporter molecule is linked thereto. The polymer backbone comprises a plurality of amine-containing acids. The compounds are useful as therapeutic and diagnostic agents, in particular in medical imaging techniques. The concept is based on the recognition that co-polymers of amino acids carrying or attached to one or more reporter molecules (e.g. chelating moieties or absorbers) are particularly suitable for diagnostic or therapeutic use. Dendrimeric polymers are the preferred backbone moieties. The compounds are formed from monomers which act as branching sites, and with each successive branching a new so-called generation is formed. The dendrimeric backbone molecule preferably comprises a multiplicity of native or non-native amino acid residues arranged so as to extend radially outwards from a central core moiety. The amino acids may be terminally bonded directly or attached via a linking group to one or more reporter groups. Alternatively, these may be terminally branched by the addition of further amino acid residues. The compounds are prepared using conventional stepwise solution synthesis. The use of di-, tri- or tetracarboxylic acids as in the present invention in the preparation is not disclosed or suggested, and, thus, the present invention is based on another principle. The structure of the resulting compounds are clearly different from the LPAs of the present invention.

As mentioned previously, the publication by Tam (ref. 2) concerns design and immunological application of MAPs. The review focuses on the recent progress achieved in MAP technology. I.a. cascade and pennant designs of core matrices for branched peptides are described including cascade and asymmetrical MAP with branching lysine, cascade and symmetrical MAP, pennant branched peptide, and polylysine as carrier. The review article does not disclose synthesis using di-, tri- or tetracarboxylic acids.

The method of the present invention is based on assembly of two identical chains attached to a solid phase by means of achiral dicarboxylic, tricarboxylic or tetracarboxylic acids. By using achiral compounds racemisation problems are avoided, thus allowing rigorous activation conditions for difficult reactions. With the exception of glycine all amino acids have a α-carbon atom including the amino dicarboxylic acids aspartic and glutamic acid. The use of chiral acids leads to racemisation, i.e. in stead of obtaining a single product, a mixture of diastereomers will be obtained, the extent varying with reaction conditions. For clinical applications, this is unacceptable and formation of clinically harmful side-products is a potential risk.

Racemisation problems arise upon activation of the carboxylic group of a chiral coupling agent, and in particular when the procedure is performed in the presence of a base, during long reaction times and/or when the reaction is carried out at elevated temperatures.

Suitable achiral di-, tri- and tetracarboxylic acids to be used in the present method have the general formula

X[(A)ₙCOOH][(B)ₘCOOH]

wherein n and m independently are an integer of from 1 to 20, X is HN, A and B independently are a substituted or unsubstituted C₁₋₁₀ alkyl, a substituted or unsubstituted C₂₋₁₀ alkenyl, a substituted or unsubstituted cyclic moiety, a substituted or unsubstituted heterocyclic moiety, or a substituted or unsubstituted aromatic moiety, or A and B together form a substituted or unsubstituted cyclic moiety, a substituted or unsubstituted heterocyclic moiety, or a substituted or unsubstituted aromatic moiety, or
n and m are 0 or an integer of from 1 to 20, X is H₂N(CR₂)ₚCR, or RHN(CR₂)ₚCR, wherein p is 0 or an integer of from 1 to 20, wherein each R is H, a substituted or unsubstituted C₁₋₁₀ alkyl, a substituted or unsubstituted C₂₋₁₀ alkenyl, a substituted or unsubstituted cyclic moiety, a substituted or unsubstituted heterocyclic moiety, or a substituted or unsubstituted aromatic moiety, and A and B are both a substituted or unsubstituted C₁₋₁₀ alkyl, a substituted or unsubstituted C₂₋₁₀ alkenyl, a substituted or unsubstituted cyclic moiety, a substituted or unsubstituted heterocyclic moiety, or A and B together form a substituted or unsubstituted cyclic moiety, a substituted or unsubstituted heterocyclic moiety, or a substituted or unsubstituted aromatic moiety, or
n and m are 0 or an integer of from 1 to 20, X is HO(CR₂)ₚCR, HS(CR₂)ₚCR, halogen-(CR₂)ₚCR, HOOC(CR₂)ₚCR, ROOC(CR₂)ₚCR, HCO(CR₂)ₚCR, RCO(CR₂)ₚCR, or [HOOC(A)ₙ]-[HOOC(B)ₘ]CR(CR₂)ₚCR, wherein p is 0 or an integer of from 1 to 20, each R independently is H, a substituted or unsubstituted C₁₋₁₀ alkyl, a substituted or unsubstituted C₂₋₁₀ alkenyl, a substituted or unsubstituted cyclic moiety, a substituted or unsubstituted heterocyclic moiety, or a substituted or unsubstituted aromatic moiety, and A and B are both a substituted or unsubstituted C₁₋₁₀ alkyl, a substituted or unsubstituted C₂₋₁₀ alkenyl, a substituted or unsubstituted cyclic moiety, a substituted or unsubstituted heterocyclic moiety, or A and B together form a substituted or unsubstituted cyclic moiety, a substituted or unsubstituted heterocyclic moiety, or a substituted or unsubstituted aromatic moiety, or
n and m are 0 or an integer of from 1 to 20, X is H₂N(CR₂)ₚ, RHN(CR₂)ₚ, HO(CR₂)ₚ, HS(CR₂)ₚ, halogen-(CR₂)ₚ, HOOC(CR₂)ₚ, ROOC(CR₂)ₚ, HCO(CR₂)ₚ, RCO(CR₂)ₚ or [HOOC(A)ₙ][HOOC(B)ₘ], wherein p is 0 or an integer of from 1 to 20, wherein each R independently is H, a substituted or unsubstituted C₁₋₁₀ alkyl, a substituted or unsubstituted C₂₋₁₀ alkenyl, a substituted or unsubstituted cyclic moiety, a substituted or unsubstituted heterocyclic moiety, or a substituted or unsubstituted aromatic moiety, and A and B together form a substituted or unsubstituted cyclic moiety, a substituted or unsubstituted heterocyclic moiety, or a substituted or unsubstituted aromatic moiety.

The term C₁₋₁₀ alkyl is intended to comprise straight or branched chain alkyl groups having 1-10 carbon atoms, e.g. methyl, ethyl, propyl, isopropyl, butyl, and tert-butyl.

The term C₂₋₁₀ alkenyl is intended to comprise straight or branched chain alkenyl groups having 2-10 carbon atoms, e.g. ethenyl, propenyl, isopropenyl, butenyl, and tert-butenyl.

The term cyclic moiety is intended to comprise e.g. cyclohexan, and cyclopentane.

The term aromatic moiety is intended to comprise e.g. phenyl.

The expression A and B forms a cyclic, heterocyclic or aromatic moiety is intended to comprise e.g. cyclohexan, piperidine, benzene, and pyridine.

By reaction with a carboxylic acid, a construct of the type

X(CO-sequence)₂-solid phase

is obtained, wherein X is as defined above.

By the term sequence, a peptide comprising naturally occurring and/or non-naturally occurring amino acids, a PNA-sequence, or a peptidomimetic is meant. Naturally occurring amino acids are L- and D-forms of the 20 amino acids found in nature. Non-naturally occurring amino acids are e.g. modified naturally occurring amino acids. The term sequence is further intended to comprise one or more of such sequences. Examples of suitable peptidomimetics are given by Marshall in ref. 21 and include oligo(N-substituted glycines), oligo carbamates, oligo-sulfones, and oligosulfoxides. Peptidomimetics also include peptoides and peptomers.

The term chemical moieties is intended to comprise an entity enhancing the solubility or immunogenecity of the LPA, an entity for directing the LPA to its target or a marker.

The group X permits directly or indirectly continued stepwise synthesis or fragment coupling of the same sequence, or of one or more different sequences and/or chemical moieties. In the case of peptides, LPAs with N to C orientation, or with simultaneous N to C and C to N presentation of the sequences are obtained. This is a unique feature of the method of the present invention. Continued synthesis or fragment coupling (fragment attachment) of a sequence different from the first such as a B or T cell epitope affords a chimeric product.

In the case where X comprises a temporarily protected amino function, synthesis or coupling can be carried out directly after deprotection. Suitable activation of all carboxy-containing groups providing effective formation of the ring system can be ensured using half equivalent carboxylic acid. In case of tri- or tetracarboxylic acids, the activated carboxy group may further be derivatised with a diamine such as ethylenediamine or an amine suitably functionalised for further reactions such as a mercapto-, an oxy-, an oxo or carboxyl group. In the case of a diamine, peptide synthesis or fragment coupling can be continued directly according to the desired sequence or chemical moiety. In a preferred embodiment, the Fmoc-protection strategy is used, but any amino protection group may be used depending on the synthesis or coupling strategy. Examples are the Boc-protection group strategy.

Since the continued stepwise synthesis or fragment coupling is performed with one or in case of a bifunctional chemical moiety such as lysine with two amino groups, it has surprisingly been found that a much better result can be obtained as compared to conventional tetrameric or octameric MAP. This is believed to be due to less interchain interaction. Furthermore, optimal peptide synthesis procedures or coupling procedures can be used for the single chains attached to the solid phase, and their homogeneity can be verified prior to forming the LPA. Cleavage from the solid phase and simultaneous side-chain deprotection can be performed by standard peptide synthesis procedures. A final LPA product may thus be obtained having optimal and well defined composition with possible by-products of less than half the molecular weight of the target molecule. Purification by standard chromatography methods such as HPLC or gel filtration can easily be performed, if desired or needed.

Favourable di-, tri- and tetracarboxylic acids for providing the ring structure are e.g. imino diacetic acid, 2-amino malonic acid, 3-amino glutaric acid, 3-methylamino glutaric acid, 3-chloro glutaric acid, 3-carboxymethyl glutaric acid, 3-methoxy-carbonyl glutaric acid, 3-acetyl glutaric acid, glutaric acid, tricarballylic acid, 3,4-bis-carboxymethyl adipic acid, 4-(2-carboxyethyl)-pimelic acid, (3,5-bis-carboxymethyl-phenyl)-acetic acid, 3,4-bis-carboxymethyl-adipic acid, benzene-1,2,4,5-tetra carboxylic acid, 4-(3-carboxy-allylamino)-but-2-enoic acid, 4,4-imino-dibenzoic acid, 1,4-dihydropyridine-3,5-dicarboxylic acid, 5-amino isophthalic acid, 2-chloro malonic acid, 3-hydroxy glutaric acid, and benzene-1,3,5-tricarboxylic acid.

In the following, the individual steps of the method according to the present invention are described in further detail. Furthermore, apart from the general procedure, specific embodiments are given.

### Step (a)

Solid phase synthesis has been known for a long time and can be carried out according to well-known standard procedures. Solid phase synthesis of peptides is e.g. described in WO 98/11125 (ref. 22). Solid phase synthesis of PNA sequences is e.g. described in WO 98/15648 (ref. 23).

Solid phase synthesis is generally carried out as described in the literature. However, a brief summary is given below.

The solid phase is preferably selected from functionalised resins such as functionalised polystyrene, polyacrylamide, polyethyleneglycol, cellulose, polyethylene, latex or dynabeads.

If desired, C-terminal amino acids may be attached to the solid support by means of a linker such as 2-Cl-trityl, 2,4-dimethoxy-4-hydroxybenzophenone, 4-(4-hydroxymethyl-3-methoxyphenoxy)-butyric acid (HMPB), 4-hydroxymethylbenzoic acid, 4-hydroxymethylphenoxyacetic acid (HMPA), 3-(4-hydroxymethylphenoxy)propionic acid or p-[(R,S)-α-[1-(9H-fluoren-9-yl)-methoxyformamido]-2,4-dimethoxybenzyl]-phenoxyacetic acid (AM).

The synthesis may be carried out batch-wise or continuously on an automated or semi-automated peptide synthesiser.

The individual coupling steps may be performed in the presence of a solvent, e.g. selected from acetonitrile, N,N-dimethylformamide (DMF), N-methylpyrrolidinone (NMP), dichloromethane (DCM), trifluoroethanol (TFE), ethanol, methanol, water, mixtures of the mentioned solvents with or without additives such as perchlorate or ethylenecarbonate.

The individual couplings between two amino acids, an amino acid and the earlier formed sequence or fragment may be carried out according to usual condensation methods such as the azide method, mixed acid anhydride method, symmetrical anhydride method, carbodiimide method, or active ester method using pentafluorophenyl (Pfp), 3,4-dihydro-4-oxobenzotriazin-3-yl (Dhbt), benzotriazol-1-yl (Bt), 7-azabenzotriazol-1-yl (At), 4-nitrophenyl, N-hydroxysuccinic acid imido esters (NHS), acid chlorides, acid fluorides, in situ activation by O-(7-azabenzotriazol-1-yl)-1,1,3,3-tetramethyluronium hexafluorophosphate (HATU), O-(benzotriazol-1-yl)-1,1,3,3-tetramethyluronium hexafluorophosphate (HBTU), O-(benzotriazol-1-yl)-1,1,3,3-tetramethyluronium tetrafluoroborate (TBTU), benzotriazolyl-oxy-tris-(dimethylamino)-phosphonium hexafluorophosphate (BOP), benzotriazolyl-N-oxytripyrrolidinophosphonium hexafluorophosphate (PyBOP), and 7-azabenzotriazolyl-N-oxytripyrrolidinophosphonium hexafluorophosphate (PyAOP).

Examples of temporary protection groups are Fmoc and Boc in the case of Cα-amino protection. Examples of side-chain protection groups are Boc, tBu, Z, and Cbz.

Examples of orthogonal protection groups are Fmoc, Boc, Mtt, Dde, All, ODmab, and Aloc.

Fmoc groups may be removed by means of an amine such as piperidine or diazabicyclo[5,4,0]undec-7-ene (DBU).

Side-chain protective groups may be removed by means of an acid such as TFA, TFMSA, HBr, HCl or HF.

To facilitate the reaction, the reaction mixture may heated to a temperature of about 60°C.

Fragment coupling (fragment coupling or fragment condensation) may be performed according to standard procedures, e.g. as described in ref. 24. Accordingly, fragments may be synthesised on a solid phase, cleaved from the solid phase with full preservation of protecting groups, purified and characterised. Synthesis of the fragments may be carried out as described above. Suitable fragments may also be obtained by other techniques including enzymatic and recombinant techniques, and coupled as described above.

### Step (b)

During solid phase synthesis or fragment coupling, N-terminal amino groups within the amino acid building blocks or side-chain amino groups of PNA moieties or peptidomimetics may be protected by suitable protecting groups. Examples of temporary protection groups are Fmoc and Boc in the case of Cα-amino protection. Examples of orthogonal protection groups are Mtt, Dde, All, ODmab, and Aloc.

If any protected amino groups are present, such may suitably be deprotected, while the sequences are still attached on the solid phase. Suitable deprotecting agents are those usually used in the peptide chemistry and include piperidine, DBU or TFA in the case of temporary protection groups, and hydrazine, Pd/AcOH/NMM, or TFA in the case of orthogonal protection groups.

### Step (c)

A ring structure is provided using a di-, tri- or tetracarboxylic acid. Formation of the ring structure is carried out under conditions enabling cyclisation of N-terminal amino groups of the desired sequences.

Suitable achiral di-, tri- and tetracarboxylic acids are given above.

Suitably, the carboxylic acid may be used in an amount of approximately 0.4 to 0.6, preferably 0.5, equivalents carboxylic acid/peptide-resin.

Cyclisation may alternatively be carried out using 1:1 orthogonal N-terminal protection. After deprotection of 0.5 eq of the peptide-chain amino-groups the di-, tri- or tetracarboxylic acid in excess is activated and coupled. After orthogonal deprotection cyclisation is achieved by activation of the second carboxylic acid group. Examples of orthogonal protection groups are Fmoc, Boc, Mtt, Dde, All, ODmab, and Aloc.

Activation may be carried out according to usual condensation methods such as the azide method, mixed acid anhydride method, symmetrical anhydride method, carbodiimide method, or active ester method using Pfp, Dhbt, Bt, At, 4-nitrophenyl, NHS esters, acid chlorides, acid fluorides, in situ activation by HATU, HBTU, TBTU, BOP, PyBOP, and PyAOP.

As already mentioned, the composition of the ligand may be verified and a sequence of the best possible purity may be achieved before the cyclisation procedure. If a test of the so obtained product is found to contain monomeric substitution product, the procedure can be repeated to achieve a possible better yield. Alternatively, in the case where free peptide chains are present after cyclisation they may be terminated by e.g. acetylation.

The reaction mixture may be heated up to about 60°C in order to facilitate the reaction. These harsh reaction conditions can be applied since an achiral carboxylic acid is used. If a chiral carboxylic acid were used, elevated reaction temperatures would promote racemisation.

### Step (d)

The resulting product, the construct, i.e. the LPA, is cleaved from the solid phase, whereby an LPA comprising ligands having free C-terminal groups are provided. Deprotection of any side-chain protecting groups can, if desired, be carried out simultaneously with cleavage from the solid phase.

Cleavage from the solid phase can be carried out using well-known agents for this purpose such as TFA, TFMSA, HBr, HCl, HF, ammonia, hydrazine, an alkoxide or a hydroxide. Alternatively, the sequence is cleaved from the solid phase by means of photolysis.

Furthermore, because the method of the invention provides an LPA of approximately the double molecular weight, standard chromatography methods such as HPLC or gel filtration can be used to obtain a single target LPA.

### Step (c¹)

If a N-protected amino group originating from the carboxylic acid used in step (c) is present, such may be deprotected. Deprotection may be carried out using well-known conditions and agents. The amino group may suitably be protected by Fmoc or Boc. Suitable deprotecting agents are those usually used in the peptide chemistry and include piperidine, and DBU or TFA.

### Step (c²)

Additional presentation of ligands comprising desired sequence(s) and/or chemical moieties may be provided by synthesis or coupling as described above.

### Dicarboxylic acids

By the deprotection in step (c¹), a free amino group is obtained. The side chains of the sequence amino acid groups are preferably still fully protected.

Synthesis or fragment coupling may then be continued on the free amino group to provide a ligand comprising one or more desired sequences, said ligand having N-protected N-terminal amino group(s). Synthesis or coupling can be carried out using peptide chemistry, synthetic procedures appropriate for PNA, or procedures appropriate for peptidomimetics. Alternatively, synthesis or coupling may be carried out so as to incorporate a chemical moiety. Such chemical moieties may suitably be a fatty acid or another compound capable of improving the solubility of the LPA or immunogenicity (adjuvant effect), an entity suitable for directing the LPA to its target, a marker, or any other group providing specific desirable properties. Lysine and amino hexanoic acid are examples of such other groups and may be used for chain extension.

Synthesis or fragment coupling may also be continued on the free amino group to provide a construct terminating with a diamino acid such as lysine, the α- and ε-amino groups of this diamino acid may be suitably protected (the protection groups being identical or different). Suitable protection groups (orthogonal protection groups) are Fmoc, Boc, Mtt, Dde, All, ODmab, and Aloc. Subsequently, solid phase synthesis or fragment coupling may be carried out on each of the amino groups (i.e. both the α- and the ε-amino group), or only on one of the amino groups (i.e. the α- or the ε-amino group) to provide a final bifunctional or trifunctional LPA. Solid phase synthesis may be carried out to provide sequences at both the α- and the ε-amino group, or only on the α-or the ε-amino group, and at the same time incorporating a chemical moiety on the other. Alternatively, chemical moieties may be incorporated on both the α- and the ε-amino group.

Examples of markers as chemical moieties are fluorophores, biotin, dinitro benzoic acid, haptens such as digoxigenin, enzymes such as HRP, alkaline phosphatases, and soya bean peroxidase, europium, and dyes such as rhodamines, cyanine dyes, coumarin, R-phycoerythrin (RPE) and allophycoerythrin.

### Tri- and tetracarboxylic acids

Prior to step (d) described above, the free carboxylic group may be activated and functionalised by use of a suitable compound containing amino functions and/or thiol and/or hydroxy functions such as 1,2-diamino ethane, 2-aminoethanthiol, and 2-aminoethanol.

In case of functionalising using a compound having a diamino function, synthesis or coupling may then be continued on the free amino group so as to provide a desired sequence having a N-protected N-terminal amino group. Synthesis can be carried out using peptide chemistry or synthesis appropriate for PNA or procedures appropriate for peptidomimetics.

If the activated and functionalised construct terminates in an amino acid such as lysine, the α- and ε-amino groups may be suitably protected (the protection groups being identical or different). Suitable protection groups (orthogonal protection groups) are Fmoc, Boc, Mtt, Dde, All, ODmab, and Aloc. Subsequently, synthesis or coupling may be carried out on each of the amino groups (i.e. both the α- and the ε-amino group), or only on one of the amino groups (i.e. the α- or the ε-amino group) so as to provide a final bifunctional or trifunctional LPA. Solid phase synthesis or fragment coupling may be carried out to provide sequences at both the α- and the ε-amino group, or only on the α- or the ε-amino group and at the same time incorporating a chemical moiety on the other. Alternatively, chemical moieties may be incorporated at both the α- and the ε-amino group. Examples of suitable chemical moieties are given above.

### Step (c³)

Deprotection of any protected amino groups may be carried out prior to, subsequently to or simultaneously with the cleavage in step (d). Suitable cleavage agents are mentioned above under step (d).

As mentioned above, a remarkable and advantageous feature by the method provides an LPA with very well-defined composition as optimised synthetic or coupling procedures can be used for preparation of the sequences.

The significant aspect of the use of amino or imino dicarboxylic acids in step (c) is the readily possibility of continued synthesis or coupling. After removal of the temporary protecting Fmoc-group in solid phase-attached and side-chain protected product, coupling with a desired activated and protected amino acid component may be effected. A typical example is attachment of Fmoc-protected amino hexanoic acid followed by standard Fmoc-deprotection with piperidine and coupling with biotin with common activating agents. The resulting product is useful for e.g. ELISA applications with avidin or streptavidin as coating agents.

The method provides i.a. LPAs presenting desired sequences with N to C orientation (using conventional terminology) (step (c)), and also simultaneously sequences with C to N orientation (in the case of incorporation of ligands comprising amino acid sequences) (step (c²)).

In a preferred embodiment of the present invention, the ligands comprising the desired sequence(s) comprise all or part of one or more B-cell epitopes, all or part of one or more T-cell epitopes, or all or part of one or more B- and T-cell epitopes (chimeras), or mimics thereof. The three-dimensional structure of a protein is often such that amino acids, which are located distant from each other in the one-dimensional structure, are located near to each other in the assembled protein. Within the meaning of the present context, the expression epitope is intended to comprise the one- and three-dimensional structure as well as mimics thereof. The term is further intended to include discontinuous B-cell epitopes.

In accordance with the present invention, the desired sequences may be derived from viruses, bacteria, toxins, allergens, autoimmune system-related agents, cancer-related agents, cell adhesion molecules, neurotropic factors, fungi or parasites.

The peptide ligands may have substitutions, deletions or additions as long as the intended function of the ligand is sufficiently preserved. By the term substitution is meant substitution of one or more amino acids with another amino acids. By the term deletion is meant removal of one or more amino acids from the sequence. By the term addition is meant insertion of one or more amino acids in the sequence. The sequence should have at least 40% identity, at least 50% identity, preferably at least 75% identity, and more preferably at least 95% identity, with the sequence from which it is derived.

In accordance with the present invention, the desired sequence may preferably be derived from viruses such as Myxoviruses e.g. influenza virus, Paramyxoviruses e.g. parainfluenza virus, mumps, measles, and Newcastle disease, Picornaviruses e.g. poliovirus, coxsackievirus, echovirus and rhinovirus, Reoviruses, Poxviruses e.g. as small pox virus, Vaccinia virus, and cowpox virus, Papovaviruses e.g. polyoma virus, papilloma virus and SV-40, Adenoviruses, Astroviruses, SRSV (small round-structures virus), Epstein-Barr virus (EBV), Parvoviruses e.g. Human Parvovirus B19, Herpesviruses e.g. herpes simplex virus, cytomegalovirus (CMV), Varicella virus (herpes zoster virus) and Pseudorabies virus, Syncytial viruses e.g. human syncytial virus (HSV), Arboviruses e.g. yellow fever, and dengue fever, Leukoviruses e.g. Rous sarcoma virus, retroviruses e.g. HIV, and Hepatitis viruses e.g. Hepatitis A, Hepatitis B and Hepatitis C,
bacteria such as Mycobacterium spp. e.g. M. tuberculosis, M. bovis, M. africanum, M. microti, M. avium, M. intracellulare, M. kansasii, M. gordonae, M. paratuberculosis, and M. lepramurium, Borrelia spp. such as B. burgdorferi, in particular B. burgdorferi sensu stricto, B. garinii, B. afzelii, B. duttoni and B. recurrentis, Salmonella spp. e.g. S. typhimurium, and S. typhi, Treponema spp. e.g. T. pallidum, Leptospira spp., Campylobacter spp. e.g. C. jejuni, Helicobacter spp. e.g. H. pylori, Pseudomonas spp., Legionella spp., Neisseria spp. e.g. N. gonorrhoea and N. menigitidis, Chlamydia spp. e.g. C. trachomatis, C. pneumonia and C. psittae, Brucella spp., Enterobacter spp., Klebsiella sppl., Yersinia spp., Vibrio spp. e.g. Vibrio chloerae, Gardnerella spp., Rickettsia spp., Clostridium spp. e.g. C. difficile and C. botulinum, Lactobacillus spp., Listeria spp., and Mycoplasma spp. e.g. M. pneumoniae and M. hominis,
toxins such as C. dificile toxin A and B, and C. tatani tetanospasmin,
allergens such as pollen allergens originating from the taxonomic order of Fagales, Oleales, Pinales, Poales, Asterales, or Urticales, a mite allergen originating from Dermatophagoides, a cockroach allergen, and animal allergen originating from cat, dog or horse, a venom allergen originating from the taxonomic order of Hymenoptera, or a fungi allergens originating from the genera Alternaria and Cladosporium,
auto-immune system-related compounds such as TNFα,
cancer-related compounds,
cell adhesion molecules,
neurotropic factors,
fungi,
parasites such Plasmodium e.g. P. falciparum, P. malariae, P. vivax, and P. ovale, Leishmania e.g. Leishmania donovani, L. brasiliensis, L. mexicana and L. peruviana, or
a sequence homologous thereto.

Two bacterial species may be of particular interest, namely Borrelia spp. and Mycobacterium spp.

The tickborne spirochete Borrelia burgdorferi is the etiological agent of Lyme borreliosis, which is at present the most common vector-borne human disease in Europe and North America.

It is desirable to have access to an assay with a high diagnostic sensitivity already in the first stage of Lyme borreliosis, in order to diagnose and treat patients before they develop severe symptoms of the later stages of Lyme borreliosis.

According to Western blot studies there are only two B. burgdorferi antigens that meet the essential criterion of eliciting an early and strong antibody response in the majority of patients. These are the B. burgdorferi flagellum and the outer surface protein C (OspC). Whereas the performance of EIAs (enzyme immuno assays) using purified native B. burgdorferi flagellum is well documented, the reported experience with OspC EIAs is still limited.

WO 97/42221 (ref. 25) discloses that the 4 terminal amino acids Pro-Lys-Lys-Pro are essential in the immune reactivity between sera from patients suffering from early borreliosis and various OspC derivatives. In order to obtain an effective diagnostic agent a polypeptide fragment which contains carboxyterminally a peptide with the general formula A⁵-A⁴-A³-A²-A¹, where A¹, and A⁴, independently from each other, designate a residue of an amino acid, wherein a nitrogen atom capable of forming part of a peptide bond is part of a ring structure; A² and A³, independently from each other, designate residues or a positively charged or polar amino acid; and A⁵ designates residues of any amino acid, such that the peptide of the formula above has a degree of sequence identity of at least 60% with the amino acid residue subsequence of Ser-Pro-Lys-Lys-Pro is preferred. Such polypeptide fragments may advantageously be used in preparing a multimeric structure according to the invention. Particular preferred OspC carboxyterminal peptides are Val-Ala-Glu-Ser-Pro-Lys-Lys-Pro, Val-Val-Ala-Glu-Ser-Pro-Lys-Lys-Pro, and Pro-Val-Val-Ala-Glu-Ser-Pro-Lys-Lys-Pro.

In one embodiment of the present method, at least one of the sequences is derived from a sequence, wherein the C-terminal amino acids are important for an immune response.

In another embodiment of the present method, the desired sequence is derived from the OspC protein of Borrelia burgdorferi, or is a homologous sequence capable of reacting with anti-OspC antibodies, or is capable of provoking an immune response. In particular, the LPA obtained provides a C-terminal presentation of the C-terminal sequence Pro-Lys-Lys-Pro of OspC.

In accordance with the invention, the presentation of a carboxyterminal OspC related peptide as described above can be combined with presentation of another sequence relevant for diagnosing Lyme borreliosis. In particular, for early diagnosis of Lyme borreliosis sequences comprising relevant epitopes from the flagellum are relevant. Other relevant sequences might be derived from OspA, OspB, OspD, OspE, OspF, Erp proteins, BmpA (P39), P100, P35, P37 and DbpA. In particular, sequences derived from OspC, the flagellum, BmpA, (P39) or P100 are suitable sequences for diagnostic uses. Sequences derived from OspA, OspC or DbpA are suitable vaccine candidates.

In yet another embodiment, the desired sequence is derived from the flagellum of Borrelia burgdorferi or is a sequence homologous thereto capable of reacting with anti-flagellum antibodies

In yet another embodiment of the present invention, the LPA providing C-terminal presentation of sequences derived from OspC of Borrelia burgdorferi further comprises desired sequence(s) derived from the flagellum of Borrelia burgdorferi.

In one embodiment of the present method, the desired sequence is derived from Mycobacterium tuberculosis. In a further embodiment, the LPA of the invention provides C-terminal presentation of the desired sequences of OspC derived from Borrelia burgdorferi, and N-terminal presentation of desired sequence(s) derived from Mycobacterium tuberculosis. In particular, the desired sequence derived from M. tuberculosis may comprise the sequence of amino acids number 51 to 70 of ESAT-6 (ESAT-6, 51-70 sequence protein) or the ESAT-6, 1-17 sequence protein of Mycobacterium tuberculosis (cf. WO 95/01441 ref. 26).

The present invention also concerns an LPA obtainable by the method defined above. The LPAs of the invention enables presentation of desired sequence(s), or presentation of desired sequence(s) and chemical moieties. Such sequences are suitably selected from a peptide comprising naturally occurring and/or non-naturally occurring amino acids, a PNA sequence, or a peptidomimetic. The chemical moieties are suitably an entity enhancing the solubility or immunogenicity of the LPA, or is an entity suitable for directing the LPA to its target, or a marker. In particular, the chemical moiety may be selected from fatty acids, antibodies or peptides for directing the LPA to its target, biotin, enzymes such as horse radish peroxidase, alkaline phosphatase and soya bean peroxidase, or nucleic acid sequences.

In one embodiment, the LPA of the invention comprises all or part of one or more B cell epitopes, all or part of one or more T cell epitopes, or all or part of one or more B and T cell epitopes, or a mimic thereof. In particular, at least one of the sequences may be derived from a sequence, wherein the C-terminal amino acids are important for an immunogenic response.

In another embodiment, the LPAs of the invention comprises sequences which are derived from viruses, bacteria, toxins, allergens, autoimmune system-related compounds, cancer related compounds, fungi or parasites, or a sequence homologous thereto.

In another embodiment, the LPAs of the invention are such, wherein the desired sequence is derived from the OspC protein of Borrelia burgdorferi. In particular, the LPA obtained may provide a C-terminal presentation of the C-terminal sequence Pro-Lys-Lys-Pro of OspC.

Examples of suitable LPAs are
[LPA-I]: FmocN(CH₂CO-ProValValAlaGluSerProLysLysPro-OH)₂ also denoted FmocN(CH₂CO-Seq. ID 1-OH)₂,
[LPA-II]: biotin-NH(CH₂)₅CON(CH₂CO-ProValValAlaGluSerPro-LysLysPro-OH)₂ also denoted biotin-NH(CH₂)₅CON(CH₂CO-Seq. ID 1-OH)₂,
[LPA-III]: NH₂CH(CH₂CO-ProValValAlaGluSerProLysLysPro-OH)₂ also denoted NH₂CH(CH₂CO-Seq. ID 1-OH)₂, and
[LPA-IV]: H-Lys-NHCH(CH₂CO-ProValValAlaGluSerProLysLys-Pro-OH)₂ also denoted H-Lys-NHCH(CH₂CO-Seq. ID 1-OH)₂.
   In another embodiment of the LPA of the present invention, the desired sequence is derived from Mycobacterium tuberculosis. In a further embodiment of the LPA, C-terminal presentation of the desired sequences derived from OspC of Borrelia burgdorferi, and N-terminal presentation of desired sequence(s) derived from Mycobacterium tuberculosis are provided.
   In particular, the LPA providing C-terminal presentation of the C-terminal Pro-Lys-Lys-Pro of OspC of Borrelia burgdorferi, and N-terminal presentation of ESAT-6 sequences from M. tuberculosis may be selected from
[LPA-V]: (HO-ProLysLysProSerGluAlaValValPro-COCH₂)₂CH-NH-Lys(GlnLeuAlaAsnAsnLeuGluThrAlaThrAlaAspTrpLysGlnGln-ValGlyGlnTyr-H)₂ also denoted (HO-Seq. ID 1-COCH₂)₂CH-NH-Lys(Seq. ID 2-H)₂, and
[LPA-VI]: (HO-ProLysLysProSerGluAlaValValPro-COCH₂)₂N-Lys(AlaSerAlaAlaAlaGluIleGlyAlaPheAsnTrpGlnGlnGluThrMet-H)₂ also denoted (HO-Seq. ID 1-COCH₂)₂N-Lys(Seq. ID 3-H)2.

Other examples of LPAs of the present invention are
[LPA-VII]: CH₂(CH₂CO-β-Ala-β-AlaLysGluProAsnLysGlyValAsn-ProAspGluValβAla)₂ also denoted CH₂(CH₂CO-β-Ala-β-Ala-Seq. ID 4-β-Ala)₂ of Chlamydia trachomatis DnaK 357-368,
[LPA-VIII]: HC(CH₂CO-LysGluProAsnLysGlyValAsnProAspGlu-ValβAla)₂COOH also denoted HC(CH₂CO-Seq. ID 4-βAla)₂COOH of Chlamydia trachomatis DnaK 357-368,
[LPA-IX]: Fmoc-NHCH(CH₂CO-AspArgValTyrIleHisProPheHisLeu-NH₂)₂ also denoted Fmoc-NHCH-(CH₂CO-Seq. ID 5-NH₂)₂ of Angiotensin-I,
[LPA-X]: Aloc-NHCH(CH₂CO-AspArgValTyrIleHisProPheHisLeu-NH₂)₂ also denoted Aloc-NHCH-(CH₂CO-Seq. ID 5-NH₂)₂ of Angiotensin-I, and
[LPA-XI]: Fmoc-AspProThrGlnAsnIleProProGly-NHCH(CH₂CO-AspArgValTyrIleHisProPheHisLeu-NH₂)₂ also denoted Fmoc-Seq. ID 6-NHCH(CH₂CO-Seq. ID 5-NH₂)₂ (Angiotensin I extended with Chlostridium thermosaccharolyticum peptide sequence 19-27).

In yet a further aspect, the present invention relates to an immunological composition for raising an immune response in an animal, including a human being comprising an LPA as defined above. In one embodiment, the immunological composition may comprise an LPA as defined above i.a. in combination with an adjuvant.

The immunological composition may suitably be in the form of a vaccine. Such a vaccine may suitably be for oral or parenteral, e.g. subcutaneous, intramuscular, intradermal, nasal, or pulmonary administration.

In another aspect, the present invention relates to the use of an LPA as defined above for preparing an immunological composition for generating antibodies in an animal, including a human being.

Furthermore, the present invention relates to the use of an LPA as defined above for preparing a pharmaceutical composition for the treatment, alleviation or prophylaxis of diseases caused by viruses, bacteria, toxins, allergens, autoimmune system-related compounds, cancer related compounds, cell adhesion molecules, neurotropic factors, fungi or parasites.

### Formulation of vaccines

Although it is preferred to insert a whole B- or T-cell epitope, it may in some cases be advantageous to insert a sequence comprising both the epitope as well as flanking regions from the protein from which the epitope is derived. The amino acids of a given epitope may be located near each other when the sequence is in the three-dimensional structure, but distant when the sequence is denatured. The sequence may be composed of the amino acids actually forming the epitope in the three-dimensional structure omitting all or part of the other amino acids.

According to the invention the LPAs may be used in vaccines against infections with pathogenic agents as described above. Strategies in formulation development of vaccines based on the products obtained by the method of the present invention generally correspond to formulation strategies for any other protein-based drug product. Potential problems and the guidance required to overcome these problems are dealt with in several textbooks. The use of an adjuvant, e.g., aluminium hydroxide, aluminium phosphate (Adju-Phos), calcium phosphate, muramyl dipeptide analogue, or some of the more recent developments in vaccine adjuvants such as biodegradable microparticles and Iscoms is a formulation challenge familiar to a pharmaceutical scientist working in this area.

Preparation of the vaccines according to the invention which contain peptide sequences as active ingredients is generally well understood in the art, as exemplified by US 4 608 251 (ref. 27), US 4 601 903 (ref. 28), US 4 599 231 (ref. 29), US 4 599 230 (ref. 30), US 4 596 792 (ref. 31), and US 4 578 770 (ref. 32), all incorporated herein by reference. Typically, such vaccines are prepared as injectables either as liquid solutions or suspensions; solid forms suitable for solution in, or suspension in, liquid prior to injection may also be prepared. The preparation may also be emulsified. The active immunogenic ingredient is often mixed with excipients which are pharmaceutically acceptable and compatible with the active ingredient. Suitable excipients are, for example, water, saline, dextrose, glycerol, ethanol, or the like, and combinations thereof. In addition, if desired, the vaccine may contain minor amounts of auxiliary substances such as wetting or emulsifying agents, pH buffering agents, or adjuvants which enhance the effectiveness of the vaccines.

The vaccines are conventionally administered parenterally, by injection, for example, either subcutaneously or intramuscularly. Additional formulations which are suitable for other modes of administration include suppositories and, in some cases, oral formulations. For suppositories, traditional binders and carriers may include, for example, polyalkylene glycols or triglycerides; such suppositories may be formed from mixtures containing the active ingredient in the range of 0.5% to 10%, preferably 1-2%. Oral formulations include such normally employed excipients as, for example, pharmaceutical grades of mannitol, lactose, starch, magnesium stearate, sodium saccharine, cellulose, magnesium carbonate, and the like. These compositions take the form of solutions, suspensions, tablets, pills, capsules, sustained release formulations or powders and contain 10-95% of active ingredient, preferably 25-70%.

The LPA according to the invention may be formulated into the vaccine as neutral or salt forms. Pharmaceutically acceptable salts include acid addition salts (formed with the free amino groups of the peptide) and which are formed with inorganic acids such as, for example, hydrochloric or phosphoric acids, or such organic acids as acetic oxalic, tartaric, mandelic, and the like. Salts formed with the free carboxyl groups may also be derived from inorganic bases such as, for example, sodium, potassium, ammonium, calcium, or ferric hydroxides, and such organic bases as isopropylamine, trimethylamine, 2-ethylamino ethanol, histidine, procaine, and the like.

The vaccines are administered in a manner compatible with the dosage formulation, and in such amount as will be therapeutically effective and immunogenic. The quantity to be administered depends on the subject to be treated, including, e.g., the capacity of the individual's immune system to mount an immune response. Suitable dosage ranges are of the order of several hundred micrograms active ingredient per vaccination with a preferred range from about 0.1 µg to 1000 µg, such as in the range from about 1 µg to 300 µg, and especially in the range from about 10 µg to 50 µg. Suitable regimens for initial administration and booster shots are also variable but are typified by an initial administration followed by subsequent inoculations or other administrations.

The manner of application may be varied widely. Any of the conventional methods for administration of a vaccine are applicable. These are believed to include oral application on a solid physiologically acceptable base or in a physiologically acceptable dispersion, parenterally, by injection or the like. The dosage of the vaccine will depend on the route of administration and will vary according to the age of the person to be vaccinated and, to a lesser degree, the size of the person to be vaccinated.

Some of the LPAs according to the present invention may be sufficiently immunogenic in a vaccine, but for some of the others the immune response will be enhanced if the vaccine further comprises an adjuvant substance.

Various methods of achieving adjuvant effect for the vaccine include use of agents such as aluminium hydroxide or aluminium phosphate (alum, Adju-Phos), commonly used as 0.05 to 0.1 percent solution in phosphate buffered saline, admixture with synthetic polymers of sugars (Carbopol) used as 0.25 percent solution, aggregation of the protein in the vaccine by heat treatment with temperatures ranging between 70°C to 101°C for 30 second to 2 minute periods respectively. Aggregation by reactivating with pepsin treated (Fab) antibodies to albumin, mixture with bacterial cells such as C. parvum or endotoxins or lipopolysaccharide components of gram-negative bacteria, emulsion in physiologically acceptable oil vehicles such as mannide mono-oleate (Aracel A) or emulsion with 20 percent solution of a perfluorocarbon (Fluosol-DA) used as a block substitute may also be employed. According to the invention DDA (dimethyldioctadecylammonium bromide) is an interesting candidate for an adjuvant, but also QuilA and RIBI are interesting possibilities. Further possibilities are monophosphoryl lipid A (MPL), and muramyl dipeptide (MDP). Other suitable adjuvants are calcium phosphate, and muramyl dipeptide analogue. Some of the more recent developments in vaccine adjuvants such as biodegradable microparticles and Iscoms may also suitably be used.

Another highly interesting possibility of achieving adjuvant effect is to employ the technique described in Gosselin *et al*., 1992 (which is hereby incorporated by reference). In brief, the presentation of a relevant antigen can be enhanced by conjugating such antigen to antibodies (or antigen binding antibody fragments) against the Fcγ receptors on monocytes/macrophages. Especially conjugates between antigen and anti-FcγRI have been demonstrated to enhance immunogenicity for the purposes of vaccination.

Other possibilities involve the use of immune modulating substances such as lymphokines (e.g. IFN-γ, IL-2 and IL-12) or synthetic IFN-γ inducers such as poly I:C in combination with the above-mentioned adjuvants.

In many instances, it will be necessary to have multiple administrations of the vaccine, usually not exceeding six vaccinations, more usually not exceeding four vaccinations and preferably one or more, usually at least about three vaccinations. The vaccinations will normally be at from two to twelve week intervals, more usually from three to five week intervals. Periodic boosters at intervals of 1-5 years, usually three years, will be desirable to maintain the desired levels of protective immunity.

One reason for admixing the LPAs of the invention with an adjuvant is to effectively activate a cellular immune response.

### Diagnosis

When the LPAs are used for diagnosis, they may be applied in various immunoassays. Samples to be tested may be obtained from e.g. body fluids such as blood, serum, cerebro spinal fluid (CSF), pleural, pericardial, synovial, pus and bone marrow, sputum, laryngeal swabs, gastric lavage, bronchial washings, biopsies, aspirates, expectorates, urine, and tissue samples and further food samples, soil, and water samples as well as cultures thereof.

Apart from the use in vaccines, the LPA of the invention may be used as a diagnostic tool. Other biochemical uses of the LPA include epitope mapping, inhibitors, artificial protein, intracellular delivery, and affinity purification of antibodies.

When the LPA is used as a diagnostic tool, a sample is brought into contact with the LPA under suitable conditions, and any reaction between the LPA and the target material is observed or measured. Suitable assays for diagnostic purposes include immunosorbent assays such as ELISA, dot blot, flow cytometry and skin tests. Such assay formats may be carried out by procedures well-known to the person skilled in the art.

The present invention further concerns a kit for use in the diagnosis of infections caused by viruses, bacteria, toxins, allergens, auto-immune system-related compounds, cancer related compounds, fungi or parasites, which kit comprises an LPA as defined above together with means for detecting or visualising binding between the LPA and the substance to be detected. In one embodiment, the kit may be for use in diagnosing diseases caused by Borrelia burgdorferi sensu lato, and comprising an LPA as defined above together with means for detecting or visualising binding between the LPA and the substance to be detected.

The present invention is further illustrated by the following non-limiting examples.

### EXAMPLES

### General apparatus

### HPLC conditions

HPLC was preformed on a Waters 600 E instrument equipped with a Waters 996 Photodiode array detector with a Waters Radial Pak 8 x 100 mm C18 reversed-phase column. Buffer A was 0.1vol % TFA in water and buffer B 90 vol% acetonitrile, 9.9 vol% water and 0.1 vol% TFA. The Buffers were pumped through the column at a flow rate of 1.5 ml/min using a linear gradient from 0% - 70% B (20 min), linear gradient from 70 - 100% B (1 min), isocratic 100% B (5 min).

### Mass spectroscopy

MALDI TOF spectra were obtained on a Fisons TofSpec E instrument. As matrix were in all cases used α-cyano-4-hydroxy-cinnamic acid. Molecular ions were recorded as M⁺H⁺ or M⁺Na⁺ and M⁺K⁺ adduct ions.

### Amino acid analysis

Amino acid analysis was performed with a Waters PICOTAG system.

### Amino acid sequencing

N-Terminal amino acid sequence determination was performed with Applied Biosystems 494 Protein Sequencer, Procise instrument.

### Peptide synthesis

### General procedures

### Apparatus and synthetic strategy

Peptides were synthesised batch-wise in a polyethylene vessel equipped with a polypropylene filter for filtration using 9-fluorenylmethyloxycarbonyl (Fmoc) or tert-butyloxycarbonyl, (Boc) as N-α-amino protecting group and suitable common protection groups for side-chain functionalities.

### Solvents

Solvent DMF (N,N-dimethylformamide, Riedel de-Häen, Germany) was purified by passing through a column packed with a strong cation exchange resin (Lewatit S 100 MB/H strong acid, Bayer AG Leverkusen, Germany) and analysed for free amines prior to use by addition of 3,4-dihydro-3-hydroxy-4-oxo-1,2,3-benzotriazine (Dhbt-OH) giving rise to a yellow colour (Dhbt-O- anion) if free amines are present. Solvent DCM (dichloromethane, analytical grade, Riedel de-Häen, Germany) was used directly without purification. Solvent NMP (N-methylpyrrolidone, Riedel de-Häen) was used without purification.

### Amino acids

Fmoc-protected amino acids and corresponding pentafluorophenyl (Pfp) esters were purchased from MilliGen (UK), NovaBiochem (Switzerland) and Bachem (Switzerland), and the Dhbt-esters from NovaBiochem (Switzerland) in suitable side-chain protected forms. Boc-protected amino acids were purchased from Bachem (Switzerland).

### Coupling reagents

Coupling reagent diisopropylcarbodiimide (DIC) was purchased from Riedel de-Häen, Germany, and destilled prior to use, dicyclohexylcarbodiimide (DCC) was purchased from Merck-Schuchardt, München, Germany, and purified by destillation. O-benzotriazolyl-N,N,N',N'-tetramethyluronium tetrafluoroborate (TBTU) was purchased from PerSeptive Biosystems GmbH Hamburg, Germany, and benzotriazolyl-N-oxy-pyrrolidinium phosphonium hexafluorophosphat (PyBOP) and 7-azabenzotriazolyl-N-oxypyrrolidinium phosphonium hexafluorophosphat (PyAOP) from PerSeptive.

### Linker

Linker 4-hydroxymethylphenoxyacetic acid (HMPA, Novabiochem, Switzerland) was coupled to the resin if required as a preformed 1-hydroxybenzotriazole (HObt) ester generated by means of DIC.

### Solid phase

Peptides synthesised according to the Fmoc-strategy were synthesised on three different types of solid support using 0.05 M or higher concentrations of Fmoc-protected activated amino acid in DMF. 1) PEG-PS (polyethyleneglycol grafted on polystyrene; TentaGel S NH2 resin, 0.27 mmol/g, TentaGel S RAM resin (s = 0.23 mmol/g), Rapp Polymere, Germany or NovaSyn TG resin, 0.29 mmol/g (denoted s), Novabiochem, Switzerland); 2) PepSyn K (Kieselguhr supported polydimethylacrylamide resin functionalised with sarcosine methyl ester 0.11 mmol/g; MilliGen, UK); 3) Chlorotrityl resin, 0.3 - 0.9 mmol/g (Novabiochem). In the two first cases the first amino acid was coupled to the resin via linker HMPA except for TentaSel S RAM which is coupled directly affording peptide amides. In the third case the first amino acid was coupled directly to the chlorotrityl resin. Peptides synthesised according to the Boc-strategy were synthesised on a Merrifield-resin (polystyrene-divinylbenzene) with the first amino acid attached (Novabiochem).

### Catalysts and other reagents

Diisopropylethylamine (DIEA), was purchased from Aldrich, Germany, ethylenediamine from Fluka, and piperidine from Riedel-de Häen, Frankfurt, Germany. 4-(N,N-dimethylamino)pyridine (DMAP) was purchased from Fluka, Switzerland and used as a catalyst in coupling reactions involving symmetrical anhydrides. 3,4-dihydro-3-hydroxy-4-oxo-1,2,3-benzotriazine (Dhbt-OH) was obtained from Fluka, Switzerland, 1-hydroxybenzotriazole (HObt) from NovaBiochem, Switzerland and 1-hydroxy-7-azabenzotriazole (HOAt) from PerSeptive. Biotin was purchased from Sigma. Scavengers Triisopropylsilan, ethanedithiol and thioanisol was purchased from Aldrich, Germany.

### General coupling procedures

The first amino acid was coupled as a symmetrical anhydride in DMF generated from the appropriate N-α-protected amino acid and DIC. The following amino acids were coupled as Pfp- or Dhbt-esters or as preformed HOBt or HOAt esters made from appropriate N-α-protected amino acids and HObt or HOAt by means of DIC or TBTU in DMF unless otherwise stated.

### Deprotection of the N-α-amino protecting group

Deprotection of the Fmoc group was performed by treatment with 20% piperidine in DMF (1x3 and 1x7 min) followed by wash with DMF until no yellow colour (Dhbt-O-) could be detected after addition of Dhbt-OH to the drained DMF. Deprotection of the Boc group was performed by treatment with 50% TFA in DCM (v/v) 1x1.5 min and 1x20 min followed by washing 6x9 min each with DCM, neutralisation with 10% triethylamine in DCM (v/v) 2x1.5 min each, followed by 6x9 min washing with DCM.

### Cleavage from solid support

Cleavage of peptide and N-terminal product from solid phase and simultaneous side-chain deprotection were done with acid. Peptides were cleaved from the resins by treatment with 95% trifluoroacetic acid (TFA, Halocarbon Products Corporation, USA; Biesterfeld & Co. Hamburg, Germany)-water v/v or with TFA (trifluoroacetic acid)-H₂O-ethanedithiole-thioanisole (90:5:3:2 vol./vol./vol./vol.) or with TFA-TIS and other scavengers at room temperature for 2 h. The filtered resins were washed with 95% TFA-water and filtrates and washings concentrated under reduced pressure. The residue was treated with ether and the precipitate freeze dried from acetic acid-water. The crude freeze dried product was analysed by HPLC and identified by MALDI-TOF MS.

### Preformed symmetrical anhydride (PSA)

6 eq. N-α-amino protected amino acid was dissolved in DCM and cooled to 0°C. DCC (3 eq.) was added and the reaction continued for 10 min to yield PSA. The solvent was removed in vacuo and the remanens dissolved in DMF. The solution was filtered and immediately added to the resin followed by 0.1 eq. of DMAP.

### Estimation of the coupling yield of the first N-α-amino protected amino acid

3-5 mg dry Fmoc-protected peptide-resin was treated with 5 ml 20% piperidine in DMF for 10 min at room temperature and the UV absorption for the dibenzofulvene-piperidine adduct was estimated at 301 nm. The yield was determined using a calculated extension coefficient e301 based on a Fmoc-Ala-OH standard.

In case of Boc-protection, the coupling was estimated according to the ninhydrin-method after removal of the Boc-group (Sarin et al., 1981, ref. 7).

### Peptide synthesis on PepSyn K resin

Dry PepSyn K (ca 500 mg), was covered by ethylenediamine and left at room temperature over night. The resin was drained and washed with DMF 10 x 15 ml, 5 min each. After draining, the resin was washed with 10% DIEA in DMF v/v (2x15 ml, 5 min each) and finally washed with DMF until no yellow colour could be detected by addition of Dhbt-OH to the drained DMF. 3 eq. HMPA 3 eq. HObt and 3 eq. DIC was dissolved in 10 ml DMF and left for activation for 10 min, after which the mixture was added to the resin and the coupling continued for 24 h. The resin was drained and washed with DMF (10x15 ml, 5 min each), and the acylation was checked by the ninhydrin test. The first amino acid was coupled as the preformed symmetrical anhydride (see above), and the coupling yields estimated as described above. It was in all cases better than 70%. The remaining amino acids according to the sequence were coupled as described above. The couplings were continued for 2 h unless otherwise specified. Excess reagent was then removed by DMF washing. After completed synthesis the peptide-resin was washed with DMF and diethyl ether and dried in vacuo.

### Peptide synthesis on PEG-PS

TentaGel S NH2 or NovaSyn TG resin (250 mg, 0.27-0.29 mmol/g) was placed in a polyethylene vessel equipped with a polypropylene filter for filtration. The resin was swelled in DMF (5ml), and treated with 20% piperidine in DMF to secure the presence of non-protonated amino groups on the resin. The resin was drained and washed with DMF until no yellow colour could be detected after addition of Dhbt-OH to the drained DMF. HMPA (3 eq.) was coupled as a preformed HObt-ester as described above and the coupling was continued for 24 h. The resin was drained and washed with DMF (5x5 ml, 5 min each) and the acylation checked by the ninhydrin test. The first amino acid was coupled as a preformed symmetrical anhydride as described above. The coupling yields of the first Fmoc-protected amino acids were estimated as described above. It was in all cases better than 70%. The following amino acids according to the sequence were coupled as preformed Fmoc-protected HObt esters (3 eq.) as described above. The couplings were continued for 2 h, unless otherwise specified. The resin was drained and washed with DMF (5x5 ml, 5 min each) in order to remove excess reagent. All acylations were checked by the ninhydrin test performed at 80 °C. After completed synthesis the peptide-resin was washed with DMF (3x5 ml, 5 min each), DCM (3x5 ml, 1 min each) and finally diethyl ether (3x5 ml, 1 min each) and dried in vacuo.

### Peptide synthesis on chlorotrityl resin

The chlorotrityl resin (approximately 0.8 mmol/g, NovaBiochem) and the first Fmoc-protected amino acid in the sequence was dried in vacuo overnight before use. Solvents, DCM and DMF, was dried over molecular sieves (4 Å, 24h) in a round bottomed flask for derivatisation dried at 100°C. The resin was placed in the flask under argon and the Fmoc-amino acid (10 eq compared to the eq of resin) was dissolved in DCM (ca. 0.5 M) with a small amount of DMF added if necessary. The solution was added to the resin followed by addition of DIEA (20 eq) and the mixture left for 30 min with stirring. The resin was transferred to a syringe fitted with a filter and washed with DCM/MeOH/DIEA (17:2:1) 3 times to cap remaining Cl, followed by washing with 3xDCM and 3xDMF. A small amount of resin, washed with DCM and MeOH prior to drying in a dessicator, was used for determination of substitution as described above by formation of dibenzofulvene-piperidine. However, treatment with piperidine was extended to at least 3-4 h prior to analysis because of slow deprotection of the Fmoc-group. After deprotection of the Fmoc-group using a prolonged deprotection, the remaining amino acids according to the sequence were coupled as described above. The couplings were continued for 2 h unless otherwise specified. Excess reagent was then removed by DMF washing. After completed synthesis the peptide-resin was washed with DMF and diethyl ether and dried in vacuo.

### Fmoc-iminodiacetic acid (Fmoc-IDA)

The product was prepared by the following method: iminodiacetic acid (2.5 g, 0.0188 mol, Aldrich) was dissolved in 10% aqueous sodium carbonate, pH 9.5 (150 ml). Fmoc-Cl (4.85 g, 0.0188 mol, Bachem) was dissolved in tetrahydrofuran (50 ml) and added dropwise over 30 min to the iminodiacetic acid solution. The mixture was left for 5 hr at room temperature, washed twice with ethyl acetate, acidified with aqueous HCl to pH 2 and Fmoc-iminodiacetic acid was extracted twice with ethylacetate. The combined extracts were washed twice with aqueous HCl (pH 2) followed by washing with brine and then concentrated in vacuo. Pentane was added to turbidity and the crystallisation was allowed to proceed for 10 min on an ice bath. The Fmoc-iminodiacetic acid was collected by filtration and the filtrate washed with cold ethylacetate/pentane and dried to give 5.61 g (84% yield). According to HPLC the purity was >99%. Mp. 212-214 °C. Literature. 213 - 215.5 °C.

### N-(Fluoren-9-ylmethoxycarbonyl)-2-aminomalonic acid (Fmoc-2-AMA)

Diethyl 2-aminomalonate (Sigma, 200 mg, 0.95 mmol) was dissolved in an aqueous solution of sodium carbonate (10%, 6 ml) and the mixture refluxed and stirred for 3h after which TLC (EtOH:AcOH (4:1) and development with ninhydrin) showed complete hydrolysis of the diethyl ester. To the reaction mixture was added 1.2 eq of O-(Fluoren-9-ylmethoxycarbonyl)-hydroxysuccinimide (NovaBiochem, Fmoc-OSu, 380 mg, 1.13 mmol) dissolved in dioxane (2 ml) over a period of 15 min and the solution stirred at room temperature overnight. Upon dilution with dioxane, the reaction mixture was acidified with HCl (1 M) to pH 5 and extracted with dichloromethane. The aqueous phase was further acidified with HCl (1 M) to pH 2 upon which the product precipitated. The aqueous phase was added ethyl acetate and stirred for 10 min. prior to extraction with ethyl acetate. The organic phase was washed with water, dried over MgSO₄ and concentrated in vacuo. The product, was crystallised from ethyl acetate - heptane with an overall yield of 135 mg (42%), mp. approximately 164°C, decomp. FAB-MS: Found 342; calculated 341.3. RP-HPLC analysis, tR 19 min, using above described conditions, revealed a minimal content of the decarboxylated product, Fmoc-Gly-OH(< 3%).

### N-(Fluoren-9-ylmethoxycarbonyl)-3-aminoglutaric acid (Fmoc-3-AGA)

3-Aminoglutaric acid (Sigma, β-glutamic acid, 30 mg, 0.20 mmol) was dissolved in 10% sodium carbonate/dioxane (1 ml/0.5 ml) and added Fmoc-OSu (83 mg, 0.25 mmol) dissolved in dioxane (1 ml) over a period of 15 min. After stirring at room temperature overnight, the reaction mixture was diluted with water and concentrated in vacuo to remove dioxane prior to extraction with DCM. The aqueous phase was separated, acidified with HCl (1 M) to pH 2-3 and the precipitated product extracted into ethyl acetate. The organic phase was washed with water, dried over MgSO₄ and concentrated in vacuo. The product was crystallised from dioxane-water yielding 47 mg (63%), mp. 189-190 °C, RP-HPLC analysis, tR 21 min using above described conditions. FAB-MS: Found 370; calculated 369.4

### Allyloxycarbonyl-3-aminoglutaric acid (Aloc-3-AGA)

3-Aminoglutaric acid (107 mg, 0.73 mmol) was dissolved in H₂O-dioxan (2.5 ml/2 ml). Triethylamine (0.2 ml, 2 eq.) and diallyl pyrocarbonate (0.12 ml, 1 eq., Lancaster, Mühlheim am Main, Germany) was added and the mixture heated under reflux over night. After evaporation of dioxan *in vacuo* the mixture was acidified with citric acid (10%, 3 ml) and extracted with ethyl acetate (3×5 ml). The organic phase was washed with water, dried over MgSO₄ and finally concentrated *in vacuo.* Trituration with pentane yielded 76 mg (45%). M.p. 117-118°C. FAB-MS: Calc. for C₉H₁₃NO₆, 231.2 found 232.0.

### 1-(4,4-Dimethyl-2,6-dioxo-cyclohex-1-ylidene)ethyl-β-Ala (Dde-β-Ala)

β-Ala (100 mg, 1.12 mmol) was dissolved in EtOH (98%, 8 ml). 2-Acetyldimedone (614 mg, 3.36 mmol, NovaBiochem) and triethylamine (0.24 ml, 3.36 mmol) was added and the mixture was refluxed for 6 hours. After continued stirring over night, the mixture was concentrated to give an oil. Dissolution in ethyl acetate (5 ml) was followed by neutralisation with citric acid (10%, 3x5 ml). The organic phase was washed with water, dried over MgSO₄ and finally concentrated *in vacuo.* Re-crystallisation from the ethyl acetate gave 180 mg (63%). M.p. 213-214°C. FAB-MS: Calc. for C₁₃H₁₉NO₄, 253.3 found 254.0.

### EXAMPLE 1

### Synthesis of FmocN(CH₂CO-ProValValAlaGluSerProLysLys-Pro)₂-chlorotrityl resin and FmocN(CH₂CO-ProValValAlaGlu-SerProLysLysPro-OH)₂ (IDA-synthesis of Seq. ID 1)

### Product: FmocN(CH₂CO-ProValValAlaGluSerProLysLysPro-OH)₂ also denoted FmocN(CH₂CO-Seq. ID 1-OH)₂ [LPA-I]

The antigenic C-terminal sequence of OspC from Borrelia burgdorferi ProValValAlaGluSerProLysLysPro (Seq. ID 1) was assembled on chlorotrityl resin (s = 0.8 mmol/g) as described above and tested for homogeneity. The peptide moiety ProValValAlaGluSerProLysLysPro has a molecular weight of 1035 Dalton and a corrected substitution of 0.44 is used for samples of the peptide-resin. 100 mg of the peptide-resin was suspended in DMF and deprotected with 20% piperidine-DMF. 0.55 eq Fmoc-iminodiacetic acid (i.e. 10% excess; 0.1x1.1x05x0.44x355 = 8.6 mg; Mw 355), 3.3 eq TBTU (0.1x1.1x3x0.44x321 = 46.6 mg; i.e. 3 times excess compared to the amount carboxy groups on Fmoc-iminodiacetic acid), 3.3 eq HObt (0.1x1.1x3x0.44x135 = 19.6) and 4.95 eq DIEA (0.1x1.1x4.5x0.44x174 = 38 ml; standard amount of DIEA is 1.5×TBTU-eq.) was added to the resin and left overnight. Excess reagents were removed, the resin was washed with DMF, followed by ether and dried. The resulting dimeric compound, FmocN(CH₂CO-ProValValAlaGluSerProLysLysPro-OH)₂, was cleaved from the resin with 95% aqueous TFA. MALDI TOF MS: Calculated for C₁₁₅H₁₇₇N₂₅O₃₂, 2421.8; found 2423.2. The product contains only a small amount of the side-product FmocN(CH₂CO-ProValValAlaGluSerProLysLysPro-OH)CH₂COOH (Calculated for C₆₇H₉₇N₁₃O₁₉, 1388.6; found 1389.0) which is essentially absent upon repeated coupling.

### EXAMPLE 2

### Synthesis of biotin-NH(CH₂)₅CON(CH₂CO-ProValValAlaGluSer-ProLysLysPro-OH)₂ (IDA derivative of Seq. ID 1 extended with 6-amino-hexanoic acid and biotinylated)

### Product: biotin-NH(CH₂)₅CON(CH₂CO-ProValValAlaGluSerPro-LysLysPro-OH)₂ also denoted Biotin-NH(CH₂)₅CON(CH₂CO-Seq. ID 1-OH)₂ [LPA-II]

FmocN(CH₂CO-ProValValAlaGluSerProLysLysPro)₂-chlorotrityl resin was deprotected with 20% piperidine-DMF and coupled with Fmoc-6-amino-hexanoic acid activated with TBTU. The coupling was followed by exhaustive acetylation with acetic anhydride. After deprotection with 20% piperidine-DMF, biotin (C₁₀H₁₆N₂O₃S) was coupled, activated with TBTU. The product was cleaved from the resin with 95% aqueous TFA. MALDI TOF MS analysis showed the target compound as the major product (calculated for C₁₁₆H₁₉₂N₂₈O₃₃S, 2539.0; found 2536.3) containing some side-product, biotin-NH(CH₂)₅CON(CH₂CO-ProValValAlaGlu-SerProLysLysPro-OH)CH₂COOH (calculated for C₆₈H₁₁₂N₁₆-O₂₀S, 1505.8; found 1504.1).

### EXAMPLE 3

### Synthesis of FmocNHCH(CH₂CO-ProValValAlaGluSerProLysLys-Pro)₂-chlorotrityl resin and NH₂CH(CH₂CO-ProValValAlaGlu-SerProLysLysPro-OH)₂ (AGA-3-derivative of Seq. ID 1)

### Product: NH₂CH(CH₂CO-ProValValAlaGluSerProLysLysPro-OH)₂ also denoted NH₂CH(CH₂CO-Seq. ID 1-OH)₂ [LPA-III]

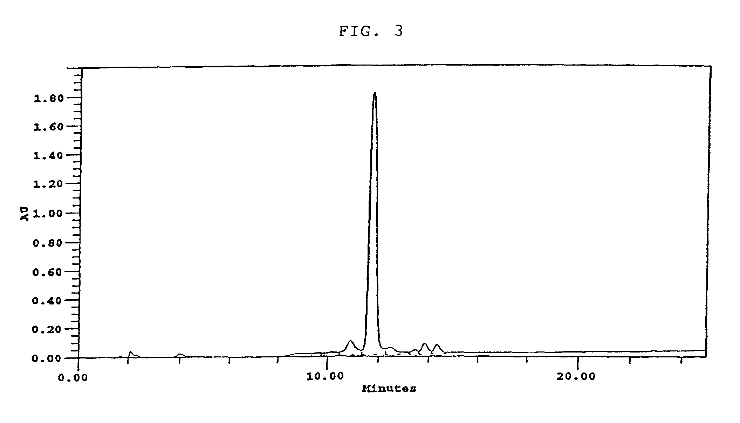

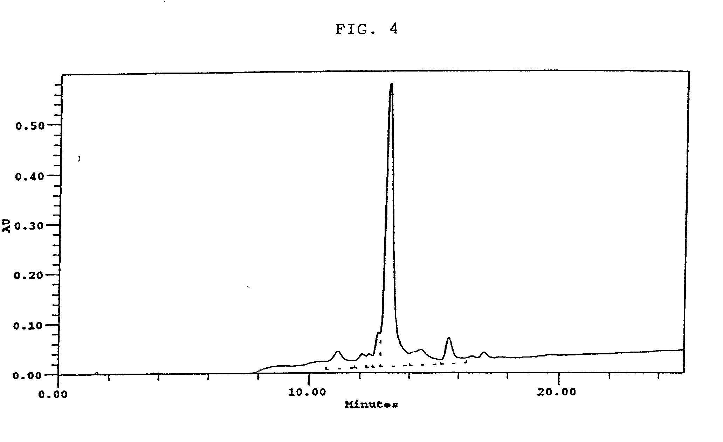

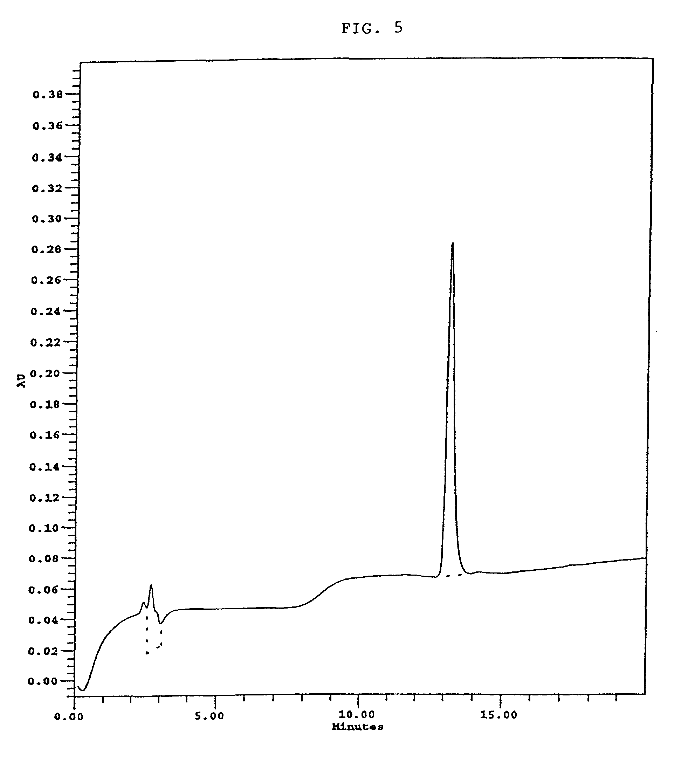

The resin derivative was assembled in the same way as described above for the iminodiacetic acid product using N-(Fluoren-9-ylmethoxycarbonyl)-3-aminoglutaric acid. The resulting Fmoc-NHCH(CH₂CO-ProValValAlaGluSerProLysLys_ Pro)₂-chlorotrityl resin was deprotected with 20% piperidine-DMF and NH₂CH(CH₂CO-ProValValAlaGluSerProLys-LysPro-OH)₂ was obtained by cleavage from the resin with 95% aqueous TFA. MALDI-TOF MS: Calculated for C₁₀₁H₁₆₉N₂₅O₃₀, 2213.6; found 2215.2 (Figures 1 and 2). The higher molecular ions are the sodium (2238.0) and potassium ion adducts (2254.3) respectively. The product contains only a very small amount of lower molecular weight products as evidenced from the spectra. HPLC of crude H-ProValValAlaGluSerProLysLysPro-OH and crude NH₂CH(CH₂CO-ProValValAlaGluSerProLysLysPro-OH)₂ is shown in Figures 3 and 4, respectively. The HPLC of the HPLC-purified LPA-III is shown in Figure 5.

### EXAMPLE 4

### Synthesis of Fmoc-Lys(Fmoc)-NHCH(CH₂CO-ProValValAlaGlu-SerProLysLysPro)₂-chlorotrityl resin and H-Lys-NHCH(CH₂-CO-ProValValAlaGluSerProLysLysPro-OH)₂ (AGA-3-derivative of Seq. ID 1 extended with di-Fmoc-lysine)

### Product: H-Lys-NHCH(CH₂CO-ProValValAlaGluSerProLysLysPro-OH)₂ also denoted H-Lys-NHCH(CH₂CO-Seq. ID 1-OH)₂ [LPA-IV]

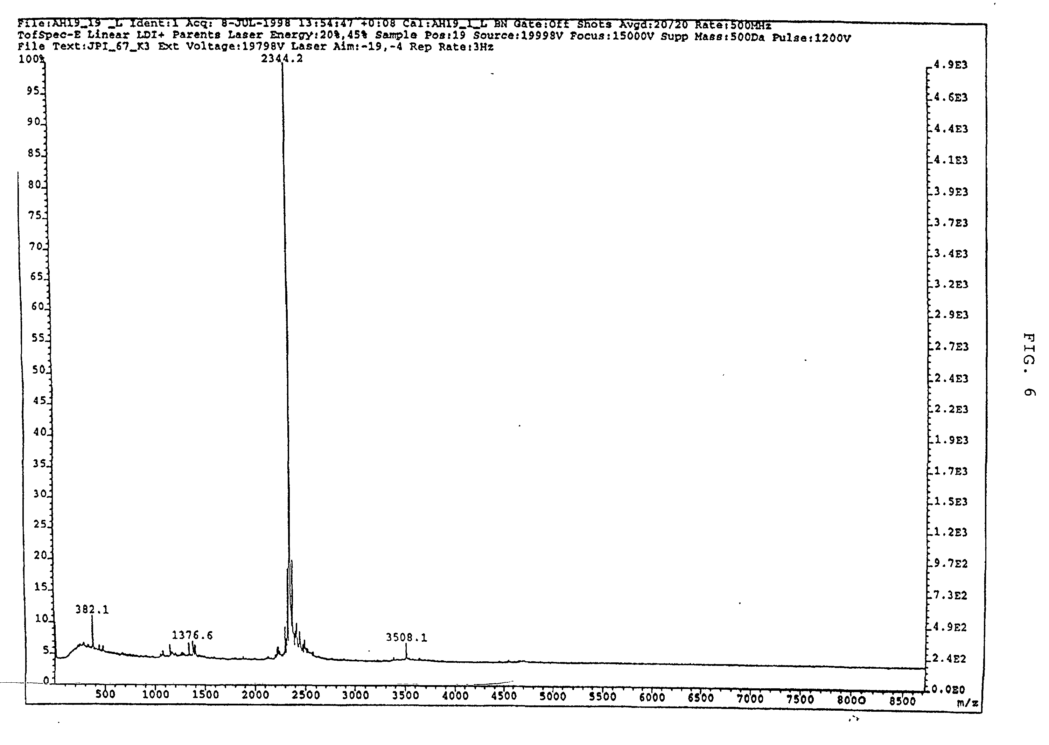

The above synthesised NH₂CH(CH₂CO-ProValValAlaGluSerPro-LysLysPro)₂-chlorotrityl resin was coupled with di-Fmoc-lysine in 3 times excess with TBTU as coupling agent. After Fmoc-deprotection with 20% piperidine-DMF, H-Lys-NHCH(CH₂CO-ProValValAlaGluSerProLysLysPro-OH)₂ was cleaved from the resin with 95% aqueous TFA and precipitated with ether. MALDI TOF MS analysis: Calculated for C₁₀₇H₁₈₁N₂₇O₃₁, 2341.8; found 2343.2 (Figure 6).

### EXAMPLE 5

### Synthesis of (HO-ProLysLysProSerGluAlaValValPro-COCH₂)₂-CH-NH-Lys(GlnLeuAlaAsnAsnLeuGluThrAlaThrAlaAspTrpLysGln-GlnValGlyGlnTyr-H)₂(AGA-3-derivative of Seq. ID 1 extended with bis-ESAT-6, 51-70 sequence (Seq. ID 2), on lysine)

### Product (HO-ProLysLysProSerGluAlaValValPro-COCH₂)₂CH-NH-Lys(GlnLeuAlaAsnAsnLeuGluThrAlaThrAlaAspTrpLysGlnGlnVal-GlyGlnTyr-H)₂ also denoted (HO-Seq. ID 1-COCH₂)₂CH-NH-Lys(Seq. ID 2-H)₂ [LPA-V]

FmocNCH(CH₂CO-ProValValAlaGluSerProLysLysPro)₂-chlorotrityl resin (55 mg; s = 0.44), was deprotected with 20% piperidine-DMF and coupled with Fmoc-Lys(Fmoc)-OH activated with TBTU to give the above Fmoc-Lys(Fmoc)-NHCH(CH₂CO-ProValValAlaGluSerProLysLysPro)₂-chlorotrityl resin. After deprotection of Fmoc-groups the amino acids according to the sequence (ESAT-6, 51-70) were coupled as the Fmoc protected amino acids using HOAt and DIC as coupling agents in 4 times excess with ≥2 h couplings followed by a double coupling of ≥30 min. After completed assembly of the peptide chains, the peptide-resin was washed with DMF and diethyl ether and dried in vacuo. The assembled product was cleaved from the resin with 95% TFA and 5% triisopropylsilane and precipitated by addition of ether, redissolved in acetic acid/water and lyophilised to give 2 mg of the target product C₃₀₅H₄₈₁N₈₃O₉₇ (Mw 6862.7). HPLC showed a major peak. The product was analysed for homogeneity by amino acid analysis. Alanine and leucine was used for comparison giving 3941 and 1937 pmol respectively corresponding to a Ala/Leu ratio of 2.04 (exp. 2.00). A double analysis similarly gave 2.03. The product was analysed by N-terminal amino acid sequencing and all 20 amino acids according to the ESAT sequence (Seq. ID 2) were recorded. The Borrelia OspC sequence (Seq. ID 1) is N-terminally coupled and consequently not sequenced.

### EXAMPLE 6

### Synthesis of (HO-ProLysLysProSerGluAlaValValPro-COCH₂)₂N-Lys(AlaSerAlaAlaAlaGluIleGlyAlaPheAsnTrpGlnGlnGluThrMet-H)2 (IDA-derivative of Seq. ID 1 extended with Seq. ID 3 on lysine)

### Product: (HO-ProLysLysProSerGluAlaValValPro-COCH₂)₂N-Lys--(AlaSerAlaAlaAlaGluIleGlyAlaPheAsnTrpGlnGlnGluThrMet-H)₂ also denoted (HO-Seq. ID 1-COCH₂)₂N-Lys(Seq. ID 3-H)₂ [LPA-VI]

FmocN(CH₂CO-ProValValAlaGluSerProLysLysPro)₂-chlorotrityl resin (55 mg; s = 0.44), was deprotected with 20% piperidine-DMF and coupled with Fmoc-Lys(Fmoc)-OH activated with TBTU to give Fmoc-Lys(Fmoc)-N(CH₂CO-Pro-ValValAlaGluSerProLysLysPro)₂-chlorotrityl resin. After deprotection of Fmoc-groups with 20% piperidine-DMF the amino acids according to the ESAT-6, 1-17 sequence were coupled as the Fmoc protected amino acids using HOAt and DIC as coupling agents in 4 times excess with ≥2 h couplings followed by a double coupling of ≥30 min. After completed assembly of the peptide chains, the peptide-resin was washed with DMF and diethyl ether and dried in vacuo. The product was cleaved from the resin with 95% TFA and 5% triisopropylsilane and precipitated by addition of ether, redissolved in acetic acid/water and lyophilised to give 8.7 mg of the target product. MALDI TOF MS analysis: Calculated for C₂₆₄H₄₀₉N₆₉O₈₃S₂, 5941.7; found 5944.7 The product was analysed for homogeneity by amino acid analysis. Proline and valine from the Borrelia pepC10 sequence was used for comparison with Gly from the ESAT-6, 1-17 sequence. Amino acid analysis gave 5044, 2823 and 1408 pmol for Pro, Val and Gly respectively giving a Gly/Pro ratio of 0.28 (exp. 0.33) and a Gly/Val ratio of 0.50 (exp. 0.500). A double analysis similarly gave 0.27 and 0.55. The product was analysed by N-terminal amino acid sequencing and all 17 amino acids according to the ESAT-6, 1-17 sequence (Seq. ID 3) were recorded. The Borrelia OspC sequence (Seq. ID 1) is N-terminally coupled and consequently not sequenced.

### EXAMPLE 7

### Synthesis of CH₂(CH₂CO-β-Ala-β-AlaLysGluProAsnLysGlyVal-AsnProAspGluValβAla)₂, (Glutaric acid-derivative, Fmoc-β-Ala and Dde-β-Ala synthesis of Seq. ID 4)

### Product: CH₂(CH₂CO-β-Ala-β-AlaLysGluProAsnLysGlyValAsn-ProAspGluValβAla)₂ also denoted CH₂(CH₂CO-β-Ala-β-Ala-Seq. ID 4-β-Ala)₂ [LPA-VII]

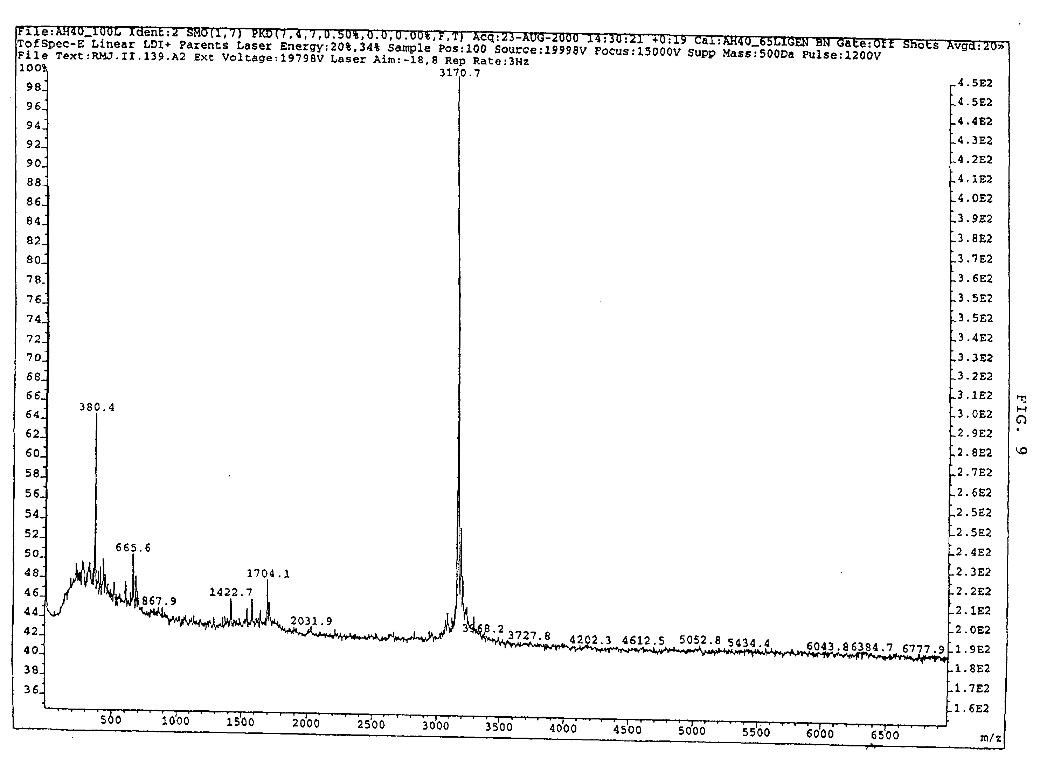

The β-Ala-β-AlaLysGluProAsnLysGlyValAsnProAspGluValβ-Ala sequence (LysGluProAsnLysGlyValAsnProAspGluVal of *Chlamydia trachomatis* DnaK 357-368) was assembled on chlorotrityl resin and on TentaGel S NH₂ resin with HMPA linker using standard coupling procedures with PyAOP 1.1 eq. and DIEA 2.2. eq. as coupling agents in DMF. In both cases the N-terminal Fmoc-α-amino group was deprotected with piperidine-DMF and subsequently coupled with a mixture of Fmoc-β-Ala and Dde-β-Ala to give an orthogonal 1:1 Fmoc/Dde-protection of the N-terminus resin-peptide chains. This was achieved by coupling with 0.75 eq. Fmoc-β-Ala and 0.25 eq. Dde-β-Ala using 1.1 eq. PyAOP and 2.2 eq. DIEA in NMP. The Fmoc-protecting group was deprotected with 20% piperidine-DMF followed by coupling with excess glutaric acid (6 eq.) and DIEA (12 eq.) in NMP over night. The remaining N-terminal Dde-α-protecting groups were deprotected with 2% hydrazine in NMP for 10 minutes, followed by washing with 5% DIEA in NMP. Assembly was then achieved over night by activation with 6 eq. PyAOP and 12 eq. DIEA in NMP. The resulting product was cleaved from the resin with TFA-H₂O-TIS (92.5:5:2.5, vol/vol/vol). A single product with the correct mass was obtained with both resins. MALDI TOF MS: Calc. for C₁₃₅H₂₁₈N₃₈O₅₀, 3173.4; found 3169.7. (trityl-resin). Figure 9 shows the MALDI-TOF MS spectrum of the product.

### EXAMPLE 8

### Synthesis of HC(CH₂CO-LysGluProAsnLysGlyValAsnProAspGlu-ValβAla)₂COOH (TCA-derivative, CH(CH₂COOH)₂COOH synthesis of Seq. ID 4)

### Product: HC(CH₂CO-LysGluProAsnLysGlyValAsnProAspGluVal--βAla)₂COOH also denoted HC(CH₂CO-Seq. ID 4-βAla)₂COOH [LPA-VIII]

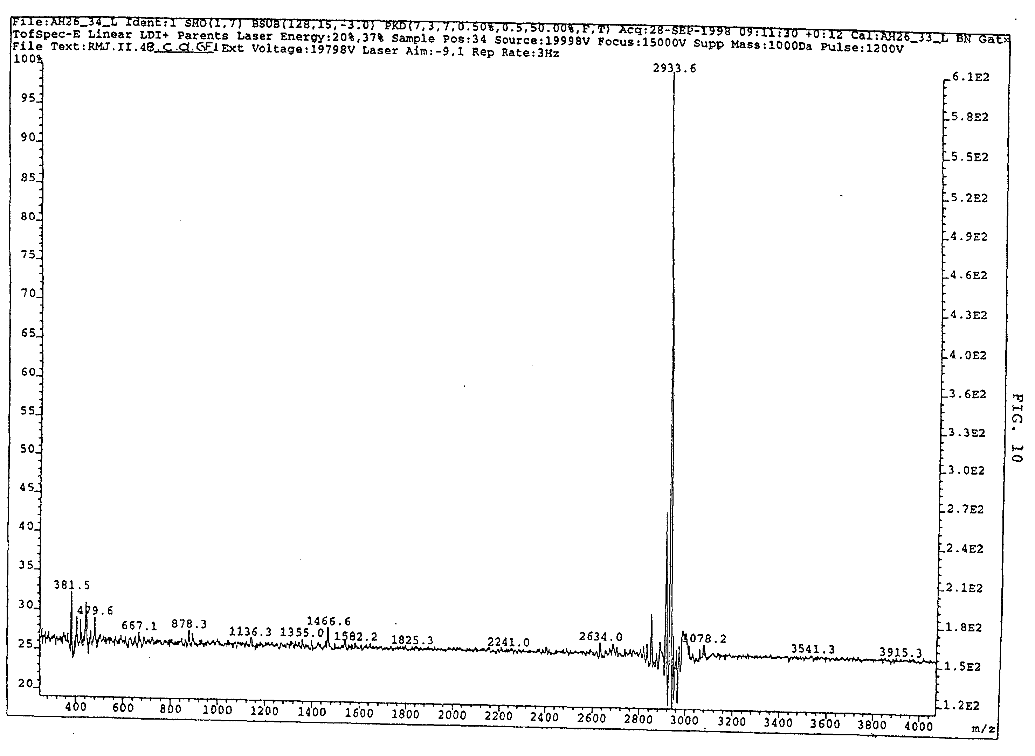

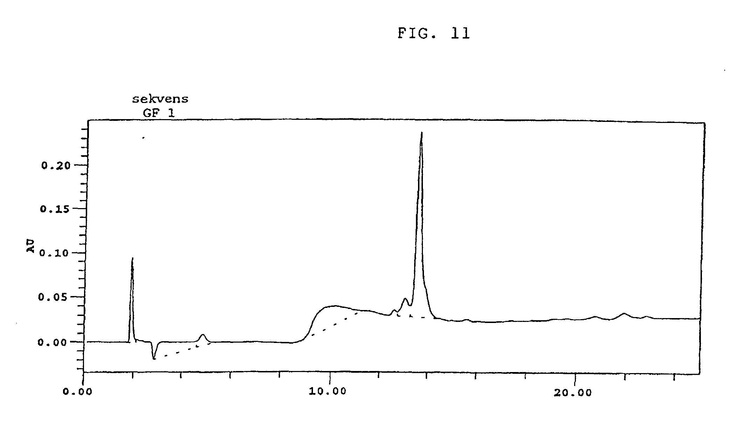

The LysGluProAsnLysGlyValAsnProAspGluValβAla sequence was assembled on TentaGel S NH₂ resin with HMPA linker (s = 0.19 mmol/g; 0.02 g) using standard coupling procedures with 3 eq. DIC and 3 eq. HOAt as coupling agents in DMF. The N-terminal Fmoc-α-amino group was deprotected with piperidine-DMF and coupled with 0.55 eq. of CH(CH₂COOH)₂COOH, 1.65 eq. PyAOP and 3.3 eq. DIEA in DMF over night. The resulting product was cleaved from the resin with TFA-H₂O-TIS-EDT (90:5:2.5:2.5, vol/vol/vol/vol) and submitted to gel filtration on G-25 Sephadex with 1% AcOH as eluent. The resulting fractions were lyophilysed to give 2 mg of the pure main product, HC(CH₂CO-LysGluProAsnLysGlyValAsnProAspGluValβAla)₂COOH (approx. 20% yield). MALDI TOF MS: Calc. for C₁₂₄H₁₉₈N₃₄O₄₈, 2933.1; found 2932.6. Figure 10 shows the MALDI-TOF MS spectrum for the product, and Figure 11 shows the HPLC chromatogram.

### EXAMPLE 9

### Synthesis of Fmoc-NHCH(CH₂CO-AspArgValTyrIleHisProPheHis-Leu-NH₂)₂, (AGA-3-derivative of angiotensin-I amide, Fmoc-3-AGA synthesis of Seq. ID 5)

### Product: Fmoc-NHCH(CH₂CO-AspArgValTyrIleHisProPheHisLeu-NH₂)₂ also denoted Fmoc-NHCH-(CH₂CO-Seq. ID 5-NH₂)₂ [LPA-IX]

The peptide sequence AspArgValTyrIleHisProPheHisLeu was assembled on TentaGel S RAM resin using standard coupling procedures with DIC and HOAt as coupling agent in DMF. The N-terminal Fmoc-α-amino group was deprotected with piperidine-DMF and coupled with 0.55 eq. Fmoc-AGA, 3.3 eqv TBTU, 3.3 eqv. HOBt and 4.95 eqv. DIEA in NMP over night. The assembled product was cleaved from the resin with TFA-H₂O-triisopropylsilane (90:5:5, vol/vol/vol) to give Fmoc-NHCH(CH₂CO-AspArgValTyrIleHisProPheHisLeu-NH₂)₂ as the major product according to MS. MALDI TOF MS: Calc. for C₁₄₄H₁₉₅N₃₇O₃₀, 2924.4; found 2923.6.

### EXAMPLE 10

### Synthesis of Fmoc-NHCH(CH₂CO-AspArgValTyrIleHisProPheHis-Leu-NH₂)₂ (AGA-3-derivative of angiotensin-I amide, Fmoc-3-AGA synthesis of Seq. ID 5)

### Product: Fmoc-NHCH(CH₂CO-AspArgValTyrIleHisProPheHisLeu-NH₂)₂ also denoted Fmoc-NHCH(CH₂CO-Seq. ID 5-NH₂)₂ [LPA-IX]

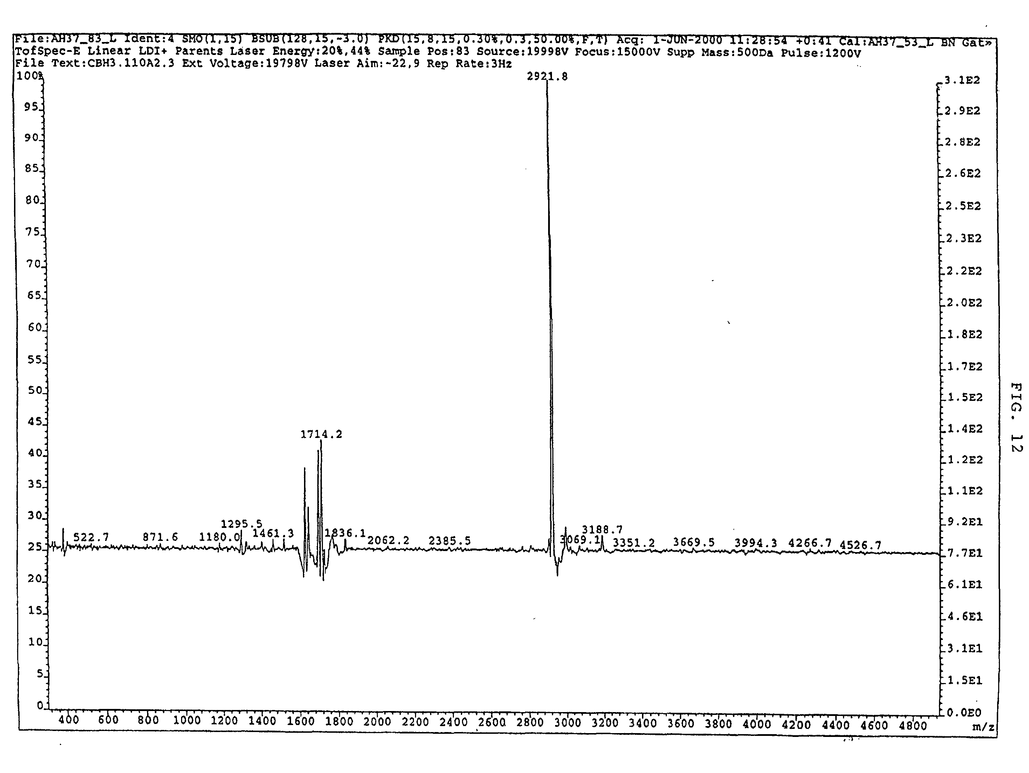

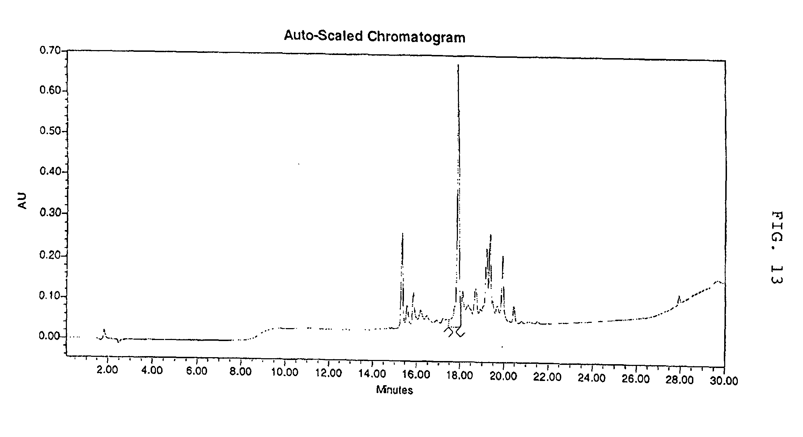

The peptide sequence AspArgValTyrIleHisProPheHisLeu was assembled on TentaGel S RAM resin (AM-linker) using standard coupling procedures with 3 eq. DIC and 3 eq. HOAt as coupling agent in DMF. The N-terminal Fmoc-α-amino group was deprotected with piperidine-DMF and coupled with 0.55 Fmoc-AGA, 3 eqv. PyAOP, 6 eqv. DIEA in DCM/NMP (3:1 vol/vol) dissolving Fmoc-AGA in NMP, PyAOP (activating agent) in DCM and adding DIEA. Coupling over night. The assembled product was cleaved from the resin with TFA-H₂O-triisopropylsilane (90:5:5, vol/vol/vol) to give Fmoc-NHCH(CH₂CO-AspArgValTyrIleHisProPheHisLeu-NH₂)₂ as the major product according to MS. MALDI TOF MS: Calc. for C₁₄₄H₁₉₅N₃₇O₃₀, 2924.4; found 2920.8. The product may be further purified by preparative HPLC or Gel filtration if desired. Figure 12 shows the MALDI-TOF MS spectrum for the crude product. Figure 13 shows the HPLC chromatogram for the crude product.

### EXAMPLE 11

### Synthesis of Aloc-NHCH(CH₂CO-AspArgValTyrIleHisProPheHis-Leu-NH₂)₂ (AGA-3-derivative of angiotensin-I amide, Fmoc-β-Ala and Dde-β-Ala synthesis of Seq. ID 5 using orthogonal protection of the sequence to be assembled)

### Product: Aloc-NHCH(CH₂CO-AspArgValTyrIleHisProPheHisLeu-NH₂)₂ also denoted Aloc-NHCH-(CH₂CO-Seq. ID 5-NH₂)₂ [LPA-X]

The peptide sequence AspArgValTyrIleHisProPheHisLeu was assembled on TentaGel S RAM resin using standard coupling procedures with 3 eq DIC and 3 eq. HOAt as coupling agents in DMF. The N-terminal Fmoc-α-amino group was deprotected with piperidine-DMF and subsequently coupled with a mixture of Fmoc-β-Ala and Dde-β-Ala to give an orthogonal 1:1 Fmoc/Dde-protection of the N-terminus resin-peptide chains. The Fmoc-protecting group was deprotected with 20% piperidine-DMF followed by over night coupling with excess Aloc-3-AGA (6 eq.) and DIEA (12 eq.) in NMP. The remaining N-terminal Dde-α-amino groups were deprotected with 2% hydrazine in NMP for 10 minutes, followed by washing with 5% DIEA in NMP. Assembly was then achieved over night by activation with 6 eq. PyAOP and 12 eq. DIEA in NMP. The resulting product was cleaved from the resin with TFA-H₂O-TIS (92.5:5:2.5, vol/vol/vol). MALDI TOF MS: Calc. for C₁₃₉H₁₉₉N₃₉O₃₂, 2928.6; found 2927.5.

### EXAMPLE 12

### Synthesis of Fmoc-AspProThrGlnAsnIleProProGly-NHCH(CH₂CO-AspArgValTyrIleHisProPheHisLeu-NH₂)₂ (AGA-3-derivative of angiotensin-I amide extended with Chlostridium thermosacchrolyticum peptide sequence 19-27)

### Product: Fmoc-AspProThrGlnAsnIleProProGly-NHCH(CH₂CO-Asp-ArgValTyrIleHisProPheHisLeu-NH₂)₂ also denoted Fmoc-Seq. ID 6-NHCH(CH₂CO-Seq. ID 5-NH₂)₂ [LPA-XI]

The Chlostridium thermosaccharolyticum peptide sequence 19-27 AspProThrGlnAsnIleProProGly was synthesised on chlorotrityl resin using standard peptide synthesis and cleaved from the resin with TFA-DCM (1:99, vol/vol) affording the fragment Fmoc-Asp(tBu)ProThr(tBu)Gln(Trt)-Asn(Trt)IleProProGly-OH. FmocNHCH(CH₂CO-AspArgValTyrIle-HisProPheHisLeu-NH₂)₂ was assembled as above (Example 11) and coupled with 3 eq. of the fragment using 3 eq. PyAOP and 6 eq. DIEA as coupling agents in NMP over night. The resulting fragment coupled product was cleaved from the resin with TFA-H2O-TIS (90:5:5, vol/vol/vol) to give the target compound together with some NH₂CH(CH₂CO-AspArgVal-TyrIleHisProPheHisLeu-NH₂)₂. MALDI TOF MS: Calc. for C₁₈₄H₂₅₄N₄₆O₄₆, 3846.3; found 3842.8.

### EXAMPLE 13

### The performance of an ELISA based on LPA-I.

The capability of LPA-I according to the present invention to react with sera from patients with neuroborreliosis was evaluated against that of an ELISA based on biotinylated-pepC10, wherein pepC10 is identical to the amino acid sequence of Seq. ID 1.

### Indirect IgM-ELISA using LPA-I and biotinylated-pepC10

### Preparing plates for the LPA-I-ELISA:

Microtiter plates (Maxisorb; Nunc, Roskilde, Denmark) were coated overnight at 4°C with 0.6 µg/ml of LPA-I diluted in 0.05 M bicarbonate pH 9.6, blocked with 3% (w/v) milk powder in phosphate-buffered saline (PBS) for 1 h, and reacted with sera diluted 1/200 in 1% (w/v) milk powder in PBS containing 0.05% Tween 20 (PBST) and 0.7 M NaCl for 2 h. The secondary antibody was a peroxidase conjugated rabbit anti-human IgM (Dako, Denmark, codes P-215) diluted 1/1000 in 1% (w/v) milk powder in PBST. After 1 h of incubation, bound secondary antibody was quantitated by colouring with o-phenylenediamine and H₂O₂ in citrate buffer (Sigma, St. Louis, Mo.) for 30 min. Optical density (OD) at 492 nm was determined in a plate reader (EAR 400 AC-SLT, Labinstruments, Austria). Plates were washed extensively with PBST-0.5M NaCl between each incubation step. The 98% specific diagnostic cut-off level was OD of 0.270 for IgM detection.

### Preparing plates for the biotinylated-pepC10-ELISA:

ELISAs with biotinylated-pepC10, were performed essentially as described above for LPA-I, except that microtiter plates were first coated with streptavidin (2.5 µg/ml) (Zymed, S. Avidinii) in citrate buffer (pH 5) overnight at 4°C, and then incubated with biotinylated pepC10 (0.1 µg/ml) in 1% (w/v) milk powder in PBST-0.37 M NaCl overnight at 4°C.

### Serum specimens from patients

### (i) Sera from 97 Danish patients with neuroborreliosis (NB).

All patients had been hospitalised in 1994 (58 males and 42 females between 4 and 80 years of age; median age, 49). The selection was based on a positive test for *B. burgdorferi* specific intrathecal antibody synthesis. The diagnosis was further corroborated by the presence of lymphocytic pleocytosis in CSF (median cell count, 124/µl) and one of the following manifestations: lymphocytic meningoradiculitis with typical painful radiculitis, mononeuritis multiplex (Bannwarths syndrome), radiculomyelitis, monosymptomatic fascialis paresis (all children), subacute lymphocytic meningitis, and chronic progressive encephalomyelitis. Of the 100 patients, 48 recalled a previous EM-like skin lesion. Disease duration, defined as the time from onset of neurological symptoms until diagnostic blood and CSF samples were taken, ranged from 1 week to 26 weeks (median duration, 3 weeks).

### Control serum specimens

Sera from 80 healthy controls were used for determination of the 98%-specific cut-off level in ELISAs.

### Results

Comparing results of the LPA-I ELISA with results of the biotinylated-pepC10 ELISA.

The following table (Table 1) shows the frequency (%) of positive Lyme borreliosis sera in the early stages of Lyme borreliosis found by the above-described LPA-I and biotinylated-pepC10 ELISAs.

**TABLE 1.**

| IgM reactivity of NB sera to biotinylated-pepC10 and LPA-I. | | |
|---|---|---|
| | No. (%) of IgM reactive sera to | |
| | biotinylated pepC10 | LPA-I |
| NB (n=97) | n=42 (43.3%) | n=41 (42.3%) |

It can be concluded that the two assays have nearly the same sensitivity.

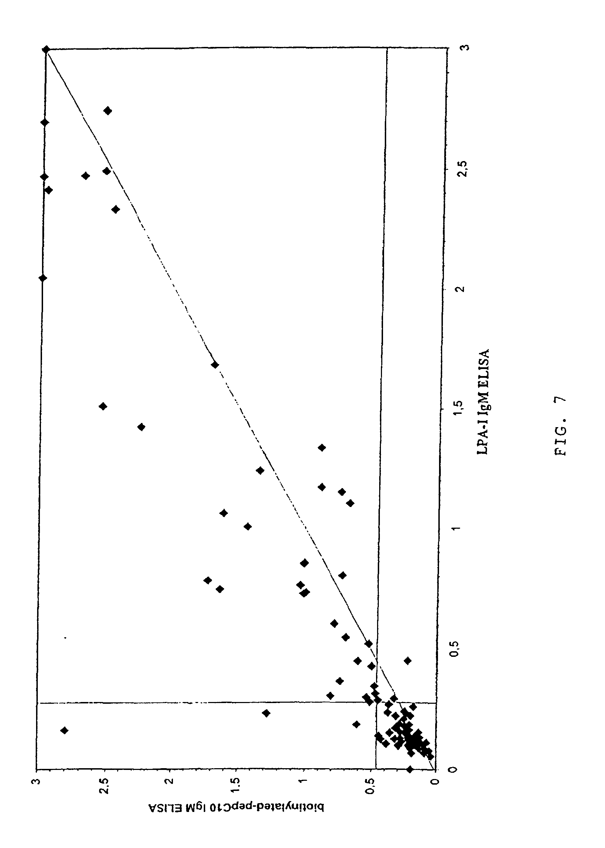

Figure 7 compares the individual results obtained in patients with NB regarding the quantitative measurement of IgM in the two ELISAs. The vertical and horizontal lines marks the 98% specific diagnostic cut-off levels for the respective LPA-I and biotinylated-pepC10 ELISA (0.275 and 0.460). As can be seen the OD titres are of the same magnitude in both assays.

It can be concluded that in this set-up, the sensitivity of the two assays are comparable. Further since the OD cut-off-value in the pepC10 ELISA is higher than the OD cut-off-value in the LPA-I ELISA it is expected that the LPA-I ELISA will perform better than the biotinylated-pepC10-ELISA when testing a large number of serum samples. Importantly, three serum samples in Figure 7 tested positive in the biotinylated-pepC10 ELISA but negative in the LPA-I ELISA. The positive reaction of all three sera is not due to anti-Borrelia antibodies but is due to the presence of anti-streptavidin antibodies which reacted with streptavidin in the biotinylated-pepC10 ELISA only. The present invention, therefore, is superior to ELISA procedures which employ peptides coupled to streptavidin because is reduces the number of false-positive serum samples.

### EXAMPLE 14

### Immunogenicity of the LPA-VI construct.

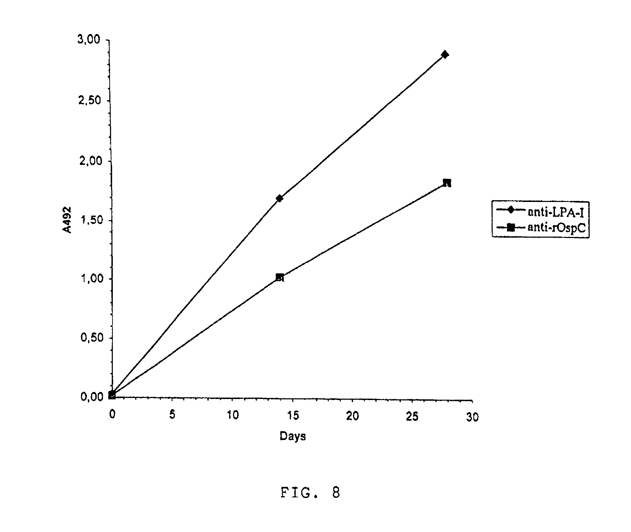

The capability of an LPA according to the present invention to induce a humoral immune response was studied by immunising mice with LPA-VI. Five female C57 black mice (age 6 to 8 weeks) were immunised two times intraperitoneally with a 2-week interval and were bled on days 0, 14, and 28. Each immunisation dose contained 5 µg of LPA-VI dissolved in 0.25 ml 0.9% NaCl and supplemented with 1 mg Al(OH)₃ (Superfos Biosector, Denmark) and 0.25 ml Freunds incomplete adjuvant. The serum samples were diluted 200 fold and then tested by ELISA for Ig reactivity against OspC produced on recombinant form (WO 97/42221) and LPA-I (Figure 8). It is concluded that LPA-VI is highly effective in inducing antibodies against the C-terminal B-cell epitope of OspC since the mice generated antibodies which recognise both recombinant OspC and LPA-I.

Figure 8. Mean ELISA titer of sera from five mice immunised with LPA-VI on days 0 and 14. The microdilution plates were coated with LPA-I (diamond) or recombinant OspC (Squares), and the sera were analysed in a 200-fold dilution.

### SEQUENCE LISTING

<110> HOLM, Arne and Statens Serum Institut
<120> Method for preparing and Ligand Presenting Assembly (LPA), and LPA, and uses thereof
<130> P199801104 WO
<140>
   <141>
<150> DK PA 1998 01233
   <151> 1998-09-29
<160> 6
<170> PatentIn Ver. 2.1
<210> 1
   <211> 10
   <212> PRT
   <213> Artificial Sequence
<220>
   <223> Description of Artificial Sequence: Sequence derived from the OspC protein of Borrelia burgdorferi
<410> 1
<210> 2
   <211> 20
   <212> PRT
   <213> Artificial Sequence
<220>
   <223> Description of Artificial Sequence: ESAT-6, 51-70 sequence of Mycobacterium tuberculosis
<400> 2
   Gln Leu Ala Asn Asn Leu Glu Thr Ala Thr Ala Asp Trp Lys Gln Gln
   1 5 10 15
   Val Gly Gln Tyr
   20
<210> 3
   <211> 17
   <212> PRT
   <213> Artificial Sequence
<220>
   <223> Description of Artificial Sequence: ESAT-6, 1-17 sequence of Mycobacterium tuberculosis
<400> 3
   Ala Ser Ala Ala Ala Glu Ile Gly Ala Phe Asn Trp Gln Gln Glu Thr
   1 5 10 15
   Met
<210> 4
   <211> 12
   <212> PRT
   <213> Artificial Sequence
<220>
   <223> Description of Artificial Sequence:Chlamydia trachomatis DnaK 357-368 sequence
<400> 4
   Lys Glu Pro Asn Lys Gly Val Asn Pro Asp Glu Val
   1 5 10
<210> 5
   <211> 10
   <212> PRT
   <213> Artificial Sequence
<220>
   <223> Description of Artificial Sequence: Angiotensin I sequence
<400> 5
   Asp Arg Val Tyr Ile His Pro Phe His Leu
   1 5 10
<210> 6
   <211> 9
   <212> PRT
   <213> Artificial Sequence
<220>
   <223> Description of Artificial Sequence: Chlostridium thermosaccharolyticum peptide sequence 19-27
<400> 6

### REFERENCES

1. PCT/US90/02039 (Tam)
2. Tam, J.P. (1996) J. Immun. Methods 196, 17-32.
3. Tomalia, D.A., Naylor, A.M., and Goddard III, W.A. (1990) Angew. Chem. Int. Ed. Engl. 29, 138-175.
4. Hodge, P. (1993) Nature (London) 362, 18-19.
5. Tam, J.P. (1995) Chapter 11, Epitope Mapping with Peptides in Peptides: Synthesis, Structures, and Applications (B. Gutte, ed.), Academic Press, 1995.
6. Heegaard, P.M.H., Bag, Y.S., Jakobsen, P.H., and Holm, A. (1997) In press.
7. Sarin et al., Anal. Biochem. 117, 147-157 (1981)
8. Nardelli et al., J. Immunol. 148, 914-920 (1992b)
9. Nardelli et al., Vaccine 12, 1335-1339 (1994)
10. Keah, H.H., Kecorius, E., and Hearn, M.T.W. (1998) J. Peptide Res. 51, 2-8.
11. Lu, Y.-A., Clavijo, P., Galantino, M., Shen, Z.-Y. and Tam, J.P. (1991) Mol. Immunol. 28, 623-630.
12. Defoort, J.P., Nardelli, B., Huang, W. and Tam, J.P. (1992) Int. J. Pept. Prot. Res. 40, 214-221.
13. Drijfhout, J. W. and Bloemhoff, W. (1991) Int. J. Pept. Prot. Res. 37, 27-32.
14. WO 93/24523 (Hafslund Nycomed)
15. Lange, M., Cuthbertson, A.S., Towart, R. and Fischer, P.M. (1998) J. Peptide Science 4, 289-293.
16. Bhatnagar, P.K., Agner, E.K., Alberts, D., Arbo, B.E., Callahan, J.F., Cuthbertson, A.S., Engelsen, S.J., Fjerdingstad, H., Hartmann, M., Heerding, D., Hiebl, J., Huffman, W.F., Hysben, M., King, A.G., Kremminger, P., Kwon, C., LoCastro, S., Løvhaug, D., Pelus, L.M., Petteway, S. and Takata, J.S., (1996) J. Med. Chem. 39, 3814-3819.
17. Alberts et al., Peptides: Chemistry, Structure and Biology (Hodges, R.S., and Smith, J.A., Eds.), 367-369 (1993), ESCOM, Leiden
18. Tuchscherer, G., and Mutter, M., Journal of Biotechnology 41, 197-210 (1995)
19. WO 94/02506 (The School of Pharmacy, University of London)
20. WO 98/32469 (Nycomed Imaging AS)
21. Marshall, G. R., Tetrahedron 49, 3547-3558 (1993)
22. WO 98/11125 (Arne Holm et al.)
23. WO 98/15648
24. Peptide Synthesis Protocols, Methods in Molecular Biology Vol. 35, Chapter 15, 303-316 in Peptide Synthesis via Fragment Condensation by R. Nyfeler, M.W. Pennington and B.M. Dunne Eds., Humana Press, 1994
25. WO 97/42221 (Statens Seruminstitut)
26. WO 95/01441 (Statens Seruminstitut)
27. US 4 608 251
28. US 4 601 903
29. US 4 599 231
30. US 4 599 230
31. US 4 596 792
32. US 4 578 770

## Claims

1. A method for preparing a ligand presenting assembly (LPA) enabling presentation of desired sequence(s) comprising the steps of
(a) providing by solid phase synthesis or fragment coupling ligands comprising desired sequence(s), the ligands being attached to a solid phase,
(b) if necessary, deprotecting any N-terminal amino groups while the ligand(s) are still attached to the solid phase,
(c) reacting the ligand(s) having unprotected N-terminal amino groups with an achiral di-, tri- or tetracarboxylic acid so as to provide a construct having a ring structure, and
(d) cleaving the construct from the solid phase so as to provide an LPA comprising ligands having free C-terminal groups.

2. A method according to claim 1 for the preparation of an LPA enabling presentation of desired sequence(s) or desired sequence(s) and chemical moieties further comprising the steps of
(c¹) if present, prior to step (d), deprotecting any N-protected amino groups originating from the carboxylic acid used in step (c),
(c²) continuing the solid phase synthesis or fragment coupling so as to provide ligand(s) comprising desired sequence(s) having at least one N-protected N-terminal amino group and/or attaching chemical moieties, and
(c³) if present, deprotecting any N-terminal amino group(s) prior to or after step (d).

3. The method according to claim 1 or 2, wherein the achiral acid used in step (c) is of the general formula
X[(A)ₙCOOH][(B)ₘCOOH]
wherein n and m independently are an integer of from 1 to 20, X is HN, A and B independently are a substituted or unsubstituted C₁₋₁₀ alkyl, a substituted or unsubstituted C₂₋₁₀ alkenyl, a substituted or unsubstituted cyclic moiety, a substituted or unsubstituted heterocyclic moiety, or a substituted or unsubstituted aromatic moiety, or A and B together form a substituted or unsubstituted cyclic moiety, a substituted or unsubstituted heterocyclic moiety, or a substituted or unsubstituted aromatic moiety, or
n and m are 0 or an integer of from 1 to 20, X is H₂N(CR₂)ₚCR, or RHN(CR₂)ₚCR, wherein p is 0 or an integer of from 1 to 20, wherein each R is H, a substituted or unsubstituted C₁₋₁₀ alkyl, a substituted or unsubstituted C₂₋₁₀ alkenyl, a substituted or unsubstituted cyclic moiety, a substituted or unsubstituted heterocyclic moiety, or a substituted or unsubstituted aromatic moiety, and A and B are both a substituted or unsubstituted C₁₋₁₀ alkyl, a substituted or unsubstituted C₂₋₁₀ alkenyl, a substituted or unsubstituted cyclic moiety, a substituted or unsubstituted heterocyclic moiety, or A and B together form a substituted or unsubstituted cyclic moiety, a substituted or unsubstituted heterocyclic moiety, or a substituted or unsubstituted aromatic moiety, or
n and m are 0 or an integer of from 1 to 20, X is HO(CR₂)ₚCR, HS(CR₂)ₚCR, halogen-(CR₂)ₚCR, HOOC(CR₂)ₚCR, ROOC(CR₂)ₚCR, HCO(CR₂)ₚCR, RCO(CR₂)ₚCR, or [HOOC(A)ₙ]-[HOOC(B)ₘ]CR(CR₂)ₚCR, wherein p is 0 or an integer of from 1 to 20, each R independently is H, a substituted or unsubstituted C₁₋₁₀ alkyl, a substituted or unsubstituted C₂₋₁₀ alkenyl, a substituted or unsubstituted cyclic moiety, a substituted or unsubstituted heterocyclic moiety, or a substituted or unsubstituted aromatic moiety, and A and B are both a substituted or unsubstituted C₁₋₁₀ alkyl, a substituted or unsubstituted C₂₋₁₀ alkenyl, a substituted or unsubstituted cyclic moiety, a substituted or unsubstituted heterocyclic moiety, or A and B together form a substituted or unsubstituted cyclic moiety, a substituted or unsubstituted heterocyclic moiety, or a substituted or unsubstituted aromatic moiety, or
n and m are 0 or an integer of from 1 to 20, X is H₂N(CR₂)ₚ, RHN(CR₂)ₚ, HO(CR₂)ₚ, HS(CR₂)ₚ, halogen-(CR₂)ₚ, HOOC(CR₂)ₚ, ROOC(CR₂)ₚ, HCO(CR₂)ₚ, RCO(CR₂)ₚ or [HOOC(A)ₙ][HOOC(B)ₘ], wherein p is 0 or an integer of from 1 to 20, each R independently is H, a substituted or unsubstituted C₁₋₁₀ alkyl, a substituted or unsubstituted C₂₋₁₀ alkenyl, a substituted or unsubstituted cyclic moiety, a substituted or unsubstituted heterocyclic moiety, or a substituted or unsubstituted aromatic moiety, and A and B together form a substituted or unsubstituted cyclic moiety, a substituted or unsubstituted heterocyclic moiety, or a substituted or unsubstituted aromatic moiety.

4. The method according to any one of claims 1-3, wherein the achiral acid is a di- or tricarboxylic acid.

5. The method according to any one of claims 1-4, wherein the achiral acid is selected among imino diacetic acid, 2-amino malonic acid, 3-amino glutaric acid, 3-methylamino glutaric acid, 3-chloro glutaric acid, 3-carboxymethyl glutaric acid, 3-methoxy-carbonyl glutaric acid, 3-acetyl glutaric acid, glutaric acid, tricarballylic acid, 3,4-bis-carboxymethyl adipic acid, 4-(2-carboxyethyl)-pimelic acid, (3,5-bis-carboxymethylphenyl)-acetic acid, 3,4-bis-carboxymethyl-adipic acid, benzene-1,2,4,5-tetra carboxylic acid, 4-(3-carboxyallylamino)-but-2-enoic acid, 4,4-imino-dibenzoic acid, 1,4-dihydropyridine-3,5-dicarboxylic acid, 5-amino isophthalic acid, 2-chloro malonic acid, 3-hydroxy glutaric acid, and benzene-1,3,5-tricarboxylic acid.

6. The method according to any one of claims 1-5, wherein the desired sequence is a peptide comprising naturally occurring amino acids and/or non-naturally occurring amino acids, a peptide nucleic acid (PNA) sequence or a peptidomimetic.

7. The method according to any one of claims 2-6, wherein the chemical moiety is an entity enhancing the solubility or immunogenicity of the LPA obtained according to claims 1-5, or is an entity suitable for directing the LPA to its target, or a marker.

8. The method according to claim 7, wherein the chemical moiety is selected from fatty acids, antibodies or peptides for directing the LPA to its target, fluorophores, biotin, enzymes such as horse radish peroxidase, alkaline phosphatase and soya bean peroxidase, or nucleic acid sequences.

9. The method according to any one of claims 1-8, wherein the desired sequence comprises all or part of one or more B cell epitopes, all or part of one or more T cell epitopes, or all or part of one or more B and T cell epitopes, or mimics thereof.

10. The method according to claim 9, wherein at least one of the sequences is derived from a sequence, wherein the C-terminal amino acids are important for an immune response.

11. The method according to any one of claims 1-10, wherein the desired sequence is derived from viruses, bacteria, toxins, allergens, autoimmune system-related compounds, cancer related compounds, cell adhesion molecules, neurotropic factors, fungi or parasites, or a sequence homologous thereto.

12. The method according to any one of claims 1-11, wherein the desired sequence is derived from OspC protein of Borrelia burgdorferi or is a homologous sequence capable of reacting with anti-OspC antibodies or of provoking an immune response.

13. The method according to claim 12, wherein the LPA obtained provide a C-terminal presentation of the C-terminal sequence Pro-Lys-Lys-Pro of OspC.

14. The method according to any one of claims 1-11, wherein the desired sequence is derived from the flagellum of Borrelia burgdorferi or is a homologous sequence capable of reacting with anti-flagellum antibodies.

15. The method according to claim 12 or 13, wherein the LPA obtained provide C-terminal presentation of sequence(s) derived from OspC of Borrelia burgdorferi and further comprises desired sequence(s) derived from the flagellum of Borrelia burgdorferi.

16. The method according to any one of claims 1-11, wherein the desired sequence is derived from Mycobacterium tuberculosis.

17. The method according to claim 12 and 13, wherein the LPA obtained further comprises desired sequence(s) derived from Mycobacterium tuberculosis.

18. The method according to claim 16 or 17, wherein the desired sequence comprises the ESAT-6, 51-70 sequence protein or the ESAT-6, 1-17 sequence protein of Mycobacterium tuberculosis.

19. An LPA obtainable by the method defined in claims 1-18.

20. An LPA according to claim 19, wherein the ligands comprising the desired sequence is selected from a peptide comprising naturally occurring and/or non-naturally occurring amino acids, a PNA sequence or a peptidomimetic.

21. An LPA according to claim 19 or 20, wherein the chemical moiety is an entity enhancing the solubility or immunogenicity of the LPA of claims 1-20 or is an entity suitable for directing the LPA to its target, or a marker.

22. An LPA according to any one of claims 19-21, wherein the chemical moiety is selected from fatty acids, antibodies or peptides for directing the LPA to its target, fluorophores, biotin, enzymes such as horse radish peroxidase, alkaline phosphatase and soya bean peroxidase, or nucleic acid sequences.

23. An LPA according to any one of claims 19-22, wherein the desired sequence comprises all or part of one or more B cell epitopes, all or part of one or more T cell epitopes, or all or part of one or more B and T cell epitopes, or mimics thereof.

24. An LPA according to claim 23, wherein at least one of the sequences is derived from a sequence, wherein the C-terminal amino acids are important for an immune response.

25. An LPA according to any one of claims 19-24, wherein the desired sequence is derived from viruses, bacteria, toxins, allergens, auto-immune system-related compounds, cancer related compounds, fungi or parasites, or a sequence homologous thereto.

26. An LPA according to any one of claims 19-24, wherein the desired sequence is derived from the OspC protein of Borrelia burgdorferi, or is a homologous sequence capable of reacting with anti-OspC antibodies or of provoking an immune response.

27. An LPA according to claim 26, wherein the LPA obtained provide a C-terminal presentation of the C-terminal sequence Pro-Lys-Lys-Pro of OspC.

28. An LPA according to any one of claims 19-27 selected from
[LPA-I]: FmocN(CH₂CO-ProValValAlaGluSerProLysLysPro-OH)₂,
[LPA-II]: biotin-NH(CH₂)₅CON(CH₂CO-ProValValAlaGluSerPro-LysLysPro-OH)₂,
[LPA-III]: NH₂CH(CH₂CO-ProValValAlaGluSerProLysLysPro-OH)₂, and
[LPA-IV]: H-Lys-NHCH(CH₂CO-ProValValAlaGluSerProLysLys-Pro-OH)₂.

29. An LPA according to any one of claims 19-25, wherein the desired sequence is derived from the flagellum of Borrelia burgdorferi or is a homologous sequence capable of reacting with anti-flagellum antibodies.

30. An LPA according to any one of claims 26-28 further comprising desired sequence(s) derived from the flagellum of Borrelia burgdorferi or is a homologous sequence capable of reacting with anti-flagellum antibodies.

31. An LPA according to any one of claims 19-25, wherein the desired sequence is derived from Mycobacterium tuberculosis.

32. An LPA according to any one of claims 26-28 further comprising desired sequence(s) derived from Mycobacterium tuberculosis.

33. An LPA according to claim 31 or 32, wherein the desired sequence comprises the ESAT-6, 51-70 sequence protein or the ESAT-6, 1-17 sequence protein of Mycobacterium tuberculosis.

34. An LPA according to any one of claims 31-33 selected from
[LPA-V]: (HO-ProLysLysProSerGluAlaValValPro-COCH₂)₂CH-NH-Lys(GlnLeuAlaAsnAsnLeuGluThrAlaThrAlaAspTrpLysGlnGln-ValGlyGlnTyr-H)₂, and
[LPA-VI]: (HO-ProLysLysProSerGluAlaValValPro-COCH₂)₂N-Lys(AlaSerAlaAlaAlaGluIleGlyAlaPheAsnTrpGlnGlnGluThrMet-H)₂.

35. An immunological composition for raising an immune response in an animal, including a human being, comprising an LPA as defined in any of claim 1-34.

36. An immunological composition according to claim 35 comprising an LPA as defined in any of claims 1-34 in combination with an adjuvant.

37. An immunological composition according to claim 35 or 36 which is in the form of a vaccine.

38. A vaccine according to claim 37 for oral or parenteral, e.g. subcutaneous, intramuscular, intradermal, nasal, or pulmonary administration.

39. Use of an LPA according to any one of claims 1 to 34 for preparing an immunological composition for generating antibodies in an animal, including a human being.

40. A kit for use in the diagnosis of infections caused by viruses, bacteria, toxins, allergens, autoimmune system-related compounds, cancer related compounds, cell adhesion molecules, neurotropic factors, fungi or parasites, which kit comprises an LPA as defined in any of claims 1-34 together with means for detecting or visualising binding between the LPA and the substance to be detected.

41. A kit for use in diagnosing diseases caused by Borrelia burgdorferi sensu lato, which kit comprises an LPA as defined in any of claims 26-30 or 32-34 together with means for detecting or visualising binding between the LPA and the substance to be detected.

42. Use of an LPA according to any one of claims 19-34 for preparing a pharmaceutical composition for the treatment, alleviation or prophylaxis of diseases caused by viruses, bacteria, toxins, allergens, autoimmune system-related compounds, cancer related compounds, cell adhesion molecules, neurotropic factors, fungi or parasites.

## Patentansprüche

1. Verfahren zur Herstellung einer Liganden präsentierenden Anordnung (ligand presenting assembly = LPA), die die Präsentation einer gewünschten Sequenz/von gewünschten Sequenzen ermöglicht, wobei das Verfahren die folgenden Schritte umfasst:
(a) Bereitstellen von Liganden, die die gewünschte(n) Sequenz(en) umfassen, durch Festphasensynthese oder Fragmentkopplung, wobei die Liganden an die Festphase gebunden sind,
(b) gegebenenfalls Entfernung der Schutzgruppen von allen N-terminalen Aminogruppen, während der Ligand/die Liganden noch an die Festphase gebunden ist/sind,
(c) Umsetzen des/der Liganden, der/die ungeschützte N-terminale Aminogruppen hat/haben, mit einer achiralen Di-, Tri- order Tetracarbonsäure zur Bereitstellung eines Konstrukts, das eine Ringstruktur hat, und
(d) Abspalten des Konstrukts von der Festphase zur Bereitstellung einer LPA, die Liganden mit freien C-terminalen Gruppen umfasst.

2. Verfahren nach Anspruch 1 zur Herstellung einer LPA, die die Präsentation einer gewünschten Sequenz/von gewünschten Sequenzenen oder einer gewünschten Sequenz/von gewünschten Sequenzen und chemischen Einheiten erlaubt, wobei das Verfahren weiterhin die folgenden Schritte umfasst:
(c¹) gegebenenfalls vor Schritt (d) Entfernen der Schutzgruppen von allen N-geschützten Aminogruppen, welche von der in Schritt (c) verwendeten Carbonsäure stammen,
(c²) Fortsetzen der Festphasensynthese oder Fragmentkopplung, um (einen) Liganden bereitzustellen, umfassend (eine) gewünschte Sequenz(en), die mindestens eine N-geschütze N-terminale Aminogruppe hat/haben, und/oder Binden von chemischen Einheiten, und
(c³) gegebenenfalls Entfernen der Schutzgruppen von (jeder) N-terminalen Aminogruppe(n) vor oder nach Schritt (d).

3. Verfahren nach Anspruch 1 oder 2, wobei die in Schritt (c) verwendete achirale Säure die allgemeine Formel
X [(A)_{*n*}COOH][(B)_{*m*}COOH]
hat, wobei n und m unabhängig eine ganze Zahl von 1 bis 20 sind, X eine HN-Gruppe ist, A und B unabhängig ein substituierter oder unsubstituierter C₁₋₁₀-Alkylrest, ein substituierter oder unsubstituierter C₂₋₁₀-Alkenylrest, eine substituierte oder unsubstituierte cyclische Einheit, eine substituierte oder unsubstituierte heterocyclische Einheit, oder eine substituierte oder unsubstituierte aromatische Einheit sind, oder A und B gemeinsam eine substituierte oder unsubstituierte cyclische Einheit, eine substituierte oder unsubstituierte heterocyclische Einheit oder eine substituierte oder unsubstituierte aromatische Einheit bilden, oder
n und m 0 oder eine ganze Zahl von 1 bis 20 sind, X der Rest H₂N(CR₂)ₚCR oder RHN(CR₂)ₚCR ist, wobei p 0 oder eine ganze Zahl von 1 bis 20 ist, wobei jeder Rest R ein Wasserstoffatom, ein substituierter oder unsubstituierter C₁₋₁₀-Alkylrest, ein substituierter oder unsubstituierter C₂₋₁₀-Alkenylrest, eine substituierte oder unsubstituierte cyclische Einheit, eine substituierte oder unsubstituierte heterocyclische Einheit, oder eine substituierte oder unsubstituierte aromatische Einheit ist, und A und B beide ein substituierter oder unsubstituierter C₁₋₁₀-Alkylrest, ein substituierter oder unsubstituierter C₂₋₁₀-Alkenylrest, eine substituierte oder unsubstituierte cyclische Einheit, eine substituierte oder unsubstituierte heterocyclische Einheit sind, oder A und B gemeinsam eine substituierte oder unsubstituierte cyclische Einheit, eine substituierte oder unsubstituierte heterocyclische Einheit oder eine substituierte oder unsubstituierte aromatische Einheit bilden, oder
n und m 0 oder eine ganze Zahl von 1 bis 20 sind, X der Rest
HO(CR₂)ₚCR, HS(CR₂)ₚCR, Halogen-(CR₂)ₚCR, HOOC(CR₂)ₚCR, ROOC(CR₂)ₚCR, HCO(CR₂)ₚCR, RCO(CR₂)ₚCR, oder [HOOC(A)ₙ]-[HOOC(B)ₘ]CR(CR₂)ₚCR ist, wobei p 0 oder eine ganze Zahl von 1 bis 20 ist, R unabhängig jeweils ein Wasserstoffatom, ein substituierter oder unsubstituierter C₁₋₁₀-Alkylrest, ein substituierter oder unsubstituierter C₂₋₁₀-Alkenylrest, eine substituierte oder unsubstituierte cyclische Einheit, eine substituierte oder unsubstituierte heterocyclische Einheit, oder eine substituierte oder unsubstituierte aromatische Einheit ist, und A und B beide ein substituierter oder unsubstituierter C₁₋₁₀-Alkylrest, ein substituierter oder unsubstituierter C₂₋₁₀-Alkenylrest, eine substituierte oder unsubstituierte cyclische Einheit, eine substituierte oder unsubstituierte heterocyclische Einheit sind, oder A und B gemeinsam eine substituierte oder unsubstituierte cyclische Einheit, eine substituierte oder unsubstituierte heterocyclische Einheit, oder eine substituierte oder unsubstituierte aromatische Einheit bilden, oder
n und m 0 oder eine ganze Zahl von 1 bis 20 sind, X der Rest
H₂N(CR₂)ₚ, RHN(CR₂)ₚ, HO(CR₂)ₚ, HS(CR₂)ₚ, Halogen-(CR₂)ₚ, HOOC(CR₂)ₚ, ROOC(CR₂)ₚ, HCO(CR₂)ₚ, RCO(CR₂)ₚ, oder [HOOC(A)ₙ] [HOOC(B)ₘ] ist, wobei p 0 oder eine ganze Zahl von 1 bis 20 ist, R unabhängig jeweils ein Wasserstoffatom, ein substituierter oder unsubstituierter C₁₋₁₀-Alkylrest, ein substituierter oder unsubstituierter C₂₋₁₀-Alkenylrest, eine substituierte oder unsubstituierte cyclische Einheit, eine substituierte oder unsubstituierte heterocyclische Einheit, oder eine substituierte oder unsubstituierte aromatische Einheit ist, und A und B gemeinsam eine substituierte oder unsubstituierte cyclische Einheit, eine substituierte oder unsubstituierte heterocyclische Einheit, oder eine substituierte oder unsubstituierte aromatische Einheit bilden.

4. Verfahren nach einem der Ansprüche 1 bis 3, wobei die achirale Säure eine Di- oder Tricarbonsäure ist.

5. Verfahren nach einem der Ansprüche 1 bis 4, wobei die achirale Säure ausgewählt ist aus Iminodiessigsäure, 2-Aminomalonsäure, 3-Aminoglutarsäure, 3-Methylaminoglutarsäure, 3-Chlorglutarsäure, 3-Carboxymethylglutarsäure, 3-Methoxycarbonylglutarsäure, 3-Acetylglutarsäure, Glutarsäure, Tricarballylsäure, 3,4-Biscarboxymethyladipinsäure, 4-(2-Carboxyethyl)-pimelinsäure, (3,5-Biscarboxymethylphenyl)-essigsäure, 3,4-Bis-carboxymethyladipinsäure, Benzol-1,2,4,5-tetracarbonsäure, 4-(3-Carboxyallylamino)-but-2-ensäure, 4,4-Imino-dibenzoesäure, 1,4-Dihydropyridin-3,5-dicarbonsäure, 5-Aminoisophthalsäure, 2-Chloromalonsäure, 3-Hydroxyglutarsäure und Benzol-1,3,5-tricarbonsäure.

6. Verfahren nach einem der Ansprüche 1 bis 5, wobei die gewünschte Sequenz ein Peptid, das natürlich vorkommende Aminosäuren und/oder nicht natürlich vorkommende Aminosäuren umfasst, eine Peptidnucleinsäure (peptide nucleic acid = PNA)-Sequenz oder ein Peptidomimetikum ist.

7. Verfahren nach einem der Ansprüche 2 bis 6, wobei die chemische Einheit eine Einheit, die die Löslichkeit oder Immunogenität der nach den Ansprüchen 1 bis 5 erhaltenen LPA verstärkt, oder eine Einheit, die die LPA an ihr Ziel führen kann, oder ein Marker ist.

8. Verfahren nach Anspruch 7, wobei die chemische Einheit ausgewählt ist aus Fettsäuren, Antikörpern oder Peptiden, die die LPA an ihr Ziel führen, Fluorophoren, Biotin, Enzymen wie z.B. Meerrettich-Peroxidase, alkalische Phosphatase und Sojabohnen-Peroxidase, oder Nucleinsäuresequenzen.

9. Verfahren nach einem der Ansprüche 1 bis 8, wobei die gewünschte Sequenz alle oder Teile von einem oder mehreren B-Zellepitopen, alle oder Teile von einem oder mehreren T-Zellepitopen, oder alle oder Teile von einem oder mehreren B- und T-Zellepitopen, oder Mimetika davon umfasst.

10. Verfahren nach Anspruch 9, wobei mindestens eine der Sequenzen von einer Sequenz abgeleitet ist, bei der die C-terminalen Aminosäuren wichtig für eine Immunantwort sind.

11. Verfahren nach einem der Ansprüche 1 bis 10, wobei die gewünschte Sequenz oder eine dazu homologe Sequenz von Viren, Bakterien, Toxinen, Allergenen, mit dem Autoimmunsystem in Zusammenhang stehenden Verbindungen, mit Krebs in Zusammenhang stehenden Verbindungen, Zelladhäsionsmolekülen, neurotropen Faktoren, Pilzen oder Parasiten, abgeleitet ist.

12. Verfahren nach einem der Ansprüche 1 bis 11, wobei die gewünschte Sequenz vom OspC-Protein von Borellia burgdorferi abgeleitet ist, oder eine homologe Sequenz ist, die mit anti-OspC-Antikörpern reagieren oder eine Immunantwort hervorrufen kann.

13. Verfahren nach Anspruch 12, wobei die erhaltene LPA eine C-terminale Präsentation der C-terminalen Sequenz Pro-Lys-Lys-Pro von OspC bereitstellt.

14. Verfahren nach einem der Ansprüche 1 bis 11, wobei die gewünschte Sequenz von dem Flagellum von Borrellia burgdorferi abgeleitet ist, oder eine homologe Sequenz ist, die mit anti-Flagellum-Antikörpern reagieren kann.

15. Verfahren nach Anspruch 12 oder 13, wobei die erhaltene LPA eine C-terminale Präsentation von (einer) Sequenz(en) bereitstellt, die von OspC von Borellia burgdorferi abgeleitet ist/sind, und weiterhin (eine) gewünschte Sequenz(en) umfasst, die von dem Flagellum von Borellia burgdorferi abgeleitet ist/sind.

16. Verfahren nach einem der Ansprüche 1 bis 11, wobei die gewünschte Sequenz von Mycobakterium tuberculosis abgeleitet ist.

17. Verfahren nach Anspruch 12 und 13, wobei die erhaltene LPA weiterhin eine gewünschte Sequenz(en) umfasst, die von Mycobacterium tuberculosis abgeleitet ist/sind.

18. Verfahren nach Anspruch 16 oder 17, wobei die gewünschte Sequenz das ESAT-6, 51-70-Sequenzprotein oder das ESAT-6, 1-17-Sequenzprotein von Mycobacterium tuberculosis umfasst.

19. LPA, erhältlich durch das in den Ansprüchen 1 bis 18 definierte Verfahren.

20. LPA nach Anspruch 19, wobei die Liganden, die die gewünschte Sequenz umfassen, ausgewählt sind aus einem Peptid, das natürlich vorkommende und/oder nicht natürlich vorkommende Aminosäuren umfasst, einer PNA-Sequenz oder einem Peptidomimetikum.

21. LPA nach Anspruch 19 oder 20, wobei die chemische Einheit eine Einheit ist, die die Löslichkeit oder Immunogenität der LPA nach den Ansprüchen 1 bis 20 verstärkt, oder eine Einheit, die die LPA an ihr Ziel führen kann, oder ein Marker ist.

22. LPA nach einem der Ansprüche 19 bis 21, wobei die chemische Einheit ausgewählt ist aus Fettsäuren, Antikörpern oder Peptiden zur Führung der LPA an ihr Ziel, Fluorophoren, Biotin, Enzymen wie z.B. Meerrettich-Peroxidase, alkalische Phosphatase und Sojabohnen-Peroxidase, oder Nucleinsäuresequenzen.

23. LPA nach einem der Ansprüche 19 bis 22, wobei die gewünschte Sequenz alle oder Teile von einem oder mehreren B-Zellepitopen, alle oder Teile von einem oder mehreren T-Zellepitopen, oder alle oder Teile von einem oder mehreren B- und T-Zellepitopen oder Mimetika davon umfasst.

24. LPA nach Anspruch 23, wobei mindestens eine der Sequenzen von einer Sequenz abgeleitet ist, bei der die C-terminalen Aminosäuren wichtig für eine Immunantwort sind.

25. LPA nach einem der Ansprüche 19 bis 24, wobei die gewünschte Sequenz oder eine dazu homologe Sequenz von Viren, Bakterien, Toxinen, Allergenen, mit dem Autoimmunsystem in Zusammenhang stehenden Verbindungen, mit Krebs in Zusammenhang stehenden Verbindungen, Pilzen oder Parasiten abgeleitet ist.

26. LPA nach einem der Ansprüche 19 bis 24, wobei die gewünschte Sequenz vom OspC-Protein von Borellia burgdorferi abgeleitet ist, oder eine homologe Sequenz ist, die mit anti-OspC-Antikörpern reagieren oder eine Immunantwort hervorrufen kann.

27. LPA nach Anspruch 26, wobei die erhaltene LPA eine C-terminale Präsentation der C-terminalen Sequenz Pro-Lys-Lys-Pro von OspC bereitstellt.

28. LPA nach einem der Ansprüche 19 bis 27, ausgewählt aus
[LPA-I]: FmocN(CH₂CO-ProValValAlaGluSerProLysLysPro-OH)₂,
[LPA-II]: Biotin-NH(CH₂)₅CON(CH₂CO-ProValValAlaGluSerProLysLysPro-OH)₂,
[LPA-III]: NH₂CH(CH₂CO-ProValValAlaGluSerProLysLysPro-OH)₂ und
[LPA-IV]: H-Lys-NHCH(CH₂CO-ProValValAlaGluSerProLysLys-Pro-OH)₂.

29. LPA nach einem der Ansprüche 19 bis 25, wobei die gewünschte Sequenz von dem Flagellum von Borrellia burgdorferi abgeleitet ist, oder eine homologe Sequenz ist, die mit anti-Flagellum-Antikörpern reagieren kann.

30. LPA nach einem der Ansprüche 26 bis 28, weiterhin umfassend die gewünschte Sequenz, die von dem Flagellum von Borellia burgdorferi abgeleitet ist, oder eine homologe Sequenz ist, die mit anti-Flagellum-Antikörpern reagieren kann.

31. LPA nach einem der Ansprüche 19 bis 25, wobei die gewünschte Sequenz von Mycobacterium tuberculosis abgleitet ist.

32. LPA nach einem der Ansprüche 26 bis 28, weiterhin umfassend die gewünschte Sequenz(en), die von Myobacterium tuberculosis abgeleitet ist/sind.

33. LPA nach Anspruch 31 oder 32, wobei die gewünschte Sequenz das ESAT-6, 51-70-Sequenzprotein oder das ESAT-6, 1-17-Sequenzprotein von Mycobacterium tuberculosis umfasst.

34. LPA nach einem der Ansprüche 31 bis 33, ausgewählt aus
[LPA-V]: (HO-ProLysLysProSerGluAlaValValPro-COCH₂)₂CH-NH-Lys(GlnLeuAlaAsnAsnLeuGluThrAlaThrAlaAspTrpLysGlnGln-ValGlyGlnTyr-H)₂, und
[LPA-VI]: (HO-ProLysLysProSerGluAlaValValPro-COCH₂)₂N-Lys(AlaSerAlaAlaAlaGlulleGlyAlaPheAsnTrpGlnGlnGluThrMet-H)₂.

35. Immunologische Zusammensetzung zum Hervorrufen einer Immunantwort in einem Tier, einschließlich des Menschen, umfassend eine wie in einem der Ansprüche 1 bis 34 definierte LPA.

36. Immunologische Zusammensetzung nach Anspruch 35, umfassend eine wie in einem der Ansprüche 1 bis 34 definierte LPA in Kombination mit einem Adjuvans.

37. Immunologische Zusammensetzung nach Anspruch 35 oder 36, die in Form eines Impfstoffes vorliegt.

38. Impfstoff nach Anspruch 37 zur oralen oder parenteralen, z.B. subkutanen, intramuskulären, intradermalen, nasalen oder pulmonalen Verabreichung.

39. Verwendung einer LPA nach einem der Ansprüche 1 bis 34 für die Herstellung einer immunologischen Zusammensetzung zur Erzeugung von Antikörpern in einem Tier, einschließlich des Menschen.

40. Kit zur Verwendung in der Diagnose von Infektionen, die durch Viren, Bakterien, Toxine, Allergene, mit dem Autoimmunsystem in Zusammenhang stehende Verbindungen, mit Krebs in Zusammenhang stehende Verbindungen, Zelladhäsionsmoleküle, neurotrope Faktoren, Pilze oder Parasiten verursacht werden, wobei der Kit eine wie in einem der Ansprüche 1 bis 34 definierte LPA zusammen mit Mitteln zum Nachweis oder Sichtbarmachen der Bindung zwischen der LPA und der nachzuweisenden Substanz umfasst.

41. Kit zur Verwendung in der Diagnose von Erkrankungen, die durch Borellia burgdorferi sensu lato hervorgerufen werden, wobei der Kit eine wie in einem der Ansprüche 26 bis 30 oder 32 bis 34 definierte LPA zusammen mit Mitteln zum Nachweis oder Sichtbarmachen der Bindung zwischen der LPA und der nachzuweisenden Substanz umfasst.

42. Verwendung einer LPA nach einem der Ansprüche 19 bis 34 für die Herstellung eines Arzneimittels zur Behandlung, Linderung oder Prophylaxe von Erkrankungen, die durch Viren, Bakterien, Toxine, Allergene, mit dem Autoimmunsystem in Zusammenhang stehende Verbindungen, mit Krebs in Zusammenhang stehende Verbindungen, Zelladhäsionsmoleküle, neurotrope Faktoren, Pilze oder Parasiten verursacht werden.

## Revendications

1. Procédé pour préparer un assemblage présentant des ligands (LPA) permettant la présentation de séquence(s) souhaitée(s) comprenant les étapes consistant à :
(a) fournir par synthèse en phase solide ou par couplage de fragments des ligands comprenant une ou des séquences souhaitées, les ligands étant liés à une phase solide ;
(b) si nécessaire, déprotéger tous groupes amino à terminaison N alors que le ou les ligands sont toujours liés à la phase solide ;
(c) faire réagir le ou les ligands ayant des groupes amino à terminaison N non protégée avec un acide di- , tri- ou tétracarboxylique achiral de façon à fournir une construction ayant une structure cyclique ; et
(d) cliver la construction à partir de la phase solide de façon à fournir un LPA comprenant des ligands ayant des groupes à terminaison C libres.

2. Procédé selon la revendication 1, pour la préparation d'un LPA permettant la présentation de séquence(s) souhaitée(s) ou de séquence(s) souhaitée(s) et de fractions chimiques comprenant de plus les étapes consistant à :
(c¹) si présents, avant l'étape (d), déprotéger tous groupes amino avec N protégé provenant de l'acide carboxylique utilisé dans l'étape (c) ;
(c²) continuer la synthèse en phase solide ou le couplage de fragments de façon à fournir un ou des ligand(s) comprenant une ou des séquences souhaitées ayant au moins un groupe amino à terminaison N avec N protégé et/ou lier des fractions chimiques ; et
(c³) si présents, déprotéger tous groupes amino à terminaison N avant ou après l'étape (d).

3. Procédé selon la revendication 1 ou 2, dans lequel l'acide achiral utilisé dans l'étape (c) est de formule générale :
X[(A)ₙCOOH][(B)ₘCOOH]
dans laquelle n et m, indépendamment l'un de l'autre, représentent un nombre entier de 1 à 20, X représente HN, A et B, indépendamment l'un de l'autre, représentent un groupe alkyle en C₁ à C₁₀ substitué ou non substitué, un groupe alcényle en C₂ à C₁₀ substitué ou non substitué, une fraction cyclique substituée ou non substituée, une fraction hétérocyclique substituée ou non substituée ou une fraction aromatique substituée ou non substituée, ou bien A et B forment ensemble une fraction cyclique substituée ou non substituée, une fraction hétérocyclique substituée ou non substituée ou une fraction aromatique substituée ou non substituée, ou
n et m valent 0 ou un nombre entier de 1 à 20, X représente H₂N(CR₂)ₚCR, ou RHN(CR₂)ₚCR, dans lequel p vaut 0 ou un nombre entier de 1 à 20, dans lequel chaque R représente H, un groupe alkyle en C₁ à C₁₀ substitué ou non substitué, un groupe alcényle en C₂ à C₁₀ substitué ou non substitué, une fraction cyclique substituée ou non substituée, une fraction hétérocyclique substituée ou non substituée ou une fraction aromatique substituée ou non substituée, et A et B représentent tous deux un groupe alkyle en C₁ à C₁₀ substitué ou non substitué, un groupe alcényle en C₂ à C₁₀ substitué ou non substitué, une fraction cyclique substituée ou non substituée, une fraction hétérocyclique substituée ou non substituée, ou bien A et B forment ensemble une fraction cyclique substituée ou non substituée, une fraction hétérocyclique substituée ou non substituée ou une fraction aromatique substituée ou non substituée, ou
n et m valent 0 ou un nombre entier de 1 à 20, X représente HO(CR₂)ₚCR, HS(CR₂)ₚCR, halogéno-(CR₂)ₚCR, HOOC(CR₂)ₚCR, ROOC(CR₂)ₚCR, HCO(CR₂)ₚCR, RCO(CR₂)ₚCR, ou [HOOC(A)ₙ]-[HOOC(B)ₘ]CR(CR₂)ₚCR, dans lequel p vaut 0 ou un nombre entier de 1 à 20, chaque R représente, indépendamment, H, un groupe alkyle en C₁ à C₁₀ substitué ou non substitué, un groupe alcényle en C₂ à C₁₀ substitué ou non substitué, une fraction cyclique substituée ou non substituée, une fraction hétérocyclique substituée ou non substituée ou une fraction aromatique substituée ou non substituée, et A et B représentent tous deux un groupe alkyle en C₁ à C₁₀ substitué ou non substitué, un groupe alcényle en C₂ à C₁₀ substitué ou non substitué, une fraction cyclique substituée ou non substituée, une fraction hétérocyclique substituée ou non substituée, ou bien A et B forment ensemble une fraction cyclique substituée ou non substituée, une fraction hétérocyclique substituée ou non substituée ou une fraction aromatique substituée ou non substituée, ou
n et m valent 0 ou un nombre entier de 1 à 20, X représente H₂N(CR₂)ₚ, RHN(CR₂)ₚ, HO(CR₂)ₚ, HS(CR₂)ₚ, halogéno-(CR₂)ₚ, HOOC(CR₂)ₚ, ROOC(CR₂)ₚ, HCO(CR₂)ₚ, RCO(CR₂)ₚ ou (HOOC(A)ₙ][HOOC(B)ₙ], dans lequel p vaut 0 ou un nombre entier de 1 à 20, chaque R représente, indépendamment, H, un groupe alkyle en C₁ à C₁₀ substitué ou non substitué, un groupe alcényle en C₂ à C₁₀ substitué ou non substitué, une fraction cyclique substituée ou non substituée, une fraction hétérocyclique substituée ou non substituée ou une fraction aromatique substituée ou non substituée, et A et B forment ensemble une fraction cyclique substituée ou non substituée, une fraction hétérocyclique substituée ou non substituée ou une fraction aromatique substituée ou non substituée.

4. Procédé selon l'une quelconque des revendications 1 à 3, dans lequel l'acide achiral est un acide di- ou tricarboxylique.

5. Procédé selon l'une quelconque des revendications 1 à 4, dans lequel l'acide achiral est choisi parmi l'acide iminodiacétique, l'acide 2-aminomalonique, l'acide 3-aminoglutarique, l'acide 3-méthylaminoglutarique, l'acide 3-chloroglutarique, l'acide 3-carboxyméthylglutarique, l'acide 3-méthoxycarbonylglutarique, l'acide 3-acétylglutarique, l'acide glutarique, l'acide tricarballylique, l'acide 3,4-bis-carboxyméthyladipique, l'acide 4-(2-carboxyéthyl)-pimélique, l'acide (3,5-bis-carboxyméthylphényl)-acétique, l'acide 3,4-bis-carboxyméthyl-adipique, l'acide benzène-1,2,4,5-tétracarboxylique, l'acide 4-(3-carboxyallylamino)-but-2-énoïque, l'acide 4,4-imino-dibenzoïque, l'acide 1,4-dihydropyridine-3,5-dicarboxylique, l'acide 5-aminoisophtalique, l'acide 2-chloromalonique, l'acide 3-hydroxyglutarique et l'acide benzène-1,3,5-tricarboxylique.

6. Procédé selon l'une quelconque des revendications 1 à 5, dans lequel la séquence souhaitée est un peptide comprenant des acides aminés d'origine naturelle et/ou des acides aminés artificiels, une séquence d'acide nucléique peptidique (PNA) ou un peptidomimétique.

7. Procédé selon l'une quelconque des revendications 2 à 6, dans lequel la fraction chimique est une entité favorisant la solubilité ou l'immunogénicité du LPA obtenu selon les revendications 1 à 5, ou bien est une entité appropriée pour diriger le LPA vers sa cible, ou un marqueur.

8. Procédé selon la revendication 7, dans lequel la fraction chimique est choisie parmi les acides gras, les anticorps ou les peptides pour diriger le LPA vers sa cible, les fluorophores, la biotine, les enzymes telles que la peroxydase de raifort, la phosphatase alcaline et la peroxidase de soja, ou des séquences d'acide nucléique.

9. Procédé selon l'une quelconque des revendications 1 à 8, dans lequel la séquence souhaitée comprend tout ou partie d'un ou plusieurs épitopes de cellules B, tout ou partie d'un ou plusieurs épitopes de cellules T, ou tout ou partie d'un ou plusieurs épitopes de cellules B et T, ou leurs mimétiques.

10. Procédé selon la revendication 9, dans lequel au moins une des séquences provient d'une séquence, dans laquelle les acides aminés à terminaison C sont importants pour une réponse immune.

11. Procédé selon l'une quelconque des revendications 1 à 10, dans lequel la séquence souhaitée provient de virus, de bactéries, de toxines, d'allergènes, de composés apparentés au système auto-immunitaire, de composés apparentés au cancer, de molécules d'adhérence cellulaire, de facteurs neurotropes, de champignons ou de parasites, ou d'une séquence homologue à ceux-ci.

12. Procédé selon l'une quelconque des revendications 1 à 11, dans lequel la séquence souhaitée provient de la protéine OspC de *Borrelia burgdorferi* ou est une séquence homologue capable de réagir avec des anticorps anti-OspC ou de provoquer une réponse immune.

13. Procédé selon la revendication 12, dans lequel le LPA obtenu fournit une présentation à terminaison C de la séquence à terminaison C, Pro-Lys-Lys-Pro de OspC.

14. Procédé selon l'une quelconque des revendications 1 à 11, dans lequel la séquence souhaitée provient de la flagelle de *Borrelia burgdorferi* ou est une séquence homologue capable de réagir avec les anticorps anti-flagelle.

15. Procédé selon la revendication 12 ou 13, dans lequel le LPA obtenu fournit une présentation à terminaison C de séquence(s) provenant de OspC de *Borrelia burgdorferi* et comprend de plus une ou des séquences souhaitées provenant de la flagelle de *Borrelia burgdorferi*.

16. Procédé selon l'une quelconque des revendications 1 à 11, dans lequel la séquence souhaitée provient de *Mycobacterium tuberculosis*.

17. Procédé selon la revendication 12 et 13, dans lequel le LPA obtenu comprend de plus une ou des séquences souhaitées provenant de *Mycobacterium tuberculosis*.

18. Procédé selon la revendication 16 ou 17, dans lequel la séquence souhaitée comprend la protéine ESAT-6, séquence 51 à 70 ou la protéine ESAT-6, séquence 1 à 17, de *Mycobacterium tuberculosis*.

19. LPA pouvant être obtenu par le procédé défini dans les revendications 1 à 18.

20. LPA selon la revendication 19, dans lequel les ligands comprenant la séquence souhaitée sont choisis à partir d'un peptide comprenant des acides aminés d'origine naturelle et/ou artificiels, une séquence PNA ou un peptidomimétique.

21. LPA selon la revendication 19 ou 20, dans lequel la fraction chimique est une entité favorisant la solubilité ou l'immunogénicité du LPA selon les revendications 1 à 20 ou est une entité appropriée pour diriger le LPA vers sa cible, ou un marqueur.

22. LPA selon l'une quelconque des revendications 19 à 21, dans lequel la fraction chimique est choisie parmi les acides gras, les anticorps ou les peptides pour diriger le LPA vers sa cible, les fluorophores, la biotine, les enzymes telles que la peroxydase de raifort, la phosphatase alcaline et la peroxidase de soja, ou les séquences d'acide nucléique.

23. LPA selon l'une quelconque des revendications 19 à 22, dans lequel la séquence souhaitée comprend tout ou partie d'un ou plusieurs épitopes de cellules B, tout ou partie d'un ou plusieurs épitopes de cellules T ou tout ou partie d'un ou plusieurs épitopes de cellules B et T, ou leurs mimétiques.

24. LPA selon la revendication 23, dans lequel au moins une des séquences provient d'une séquence, dans laquelle les acides aminés à terminaison C sont importants pour une réponse immune.

25. LPA selon l'une quelconque des revendications 19 à 24, dans lequel la séquence souhaitée provient de virus, de bactéries, de toxines, d'allergènes, de composés apparentés au système auto-immunitaire, de composés apparentés au cancer, de champignons ou de parasites, ou d'une séquence homologue à ceux-ci.

26. LPA selon l'une quelconque des revendications 19 à 24, dans lequel la séquence souhaitée provient de la protéine OspC de *Borrelia burgdorferi*, ou est une séquence homologue capable de réagir avec les anticorps anti-OspC ou de provoquer une réponse immune.

27. LPA selon la revendication 26, dans lequel le LPA obtenu fournit une présentation à terminaison C de la séquence à terminaison C, Pro-Lys-Lys-Pro de OspC.

28. LPA selon l'une quelconque des revendications 19 à 27, choisi parmi :
[LPA-I] : FmocN(CH₂CO-ProValValAlaGluSerProLysLysPro-OH)₂,
[LPA-II] : biotine-NH(CH₂)₅CON(CH₂CO-ProValValAlaGluSerPro-LysLysPro-OH)₂,
[LPA-III] : NH₂CH(CH₂CO-ProValValAlaGluSerProLysLysPro-OH)₂, et
[LPA-IV] : H-Lys-NHCH(CH₂CO-ProValValAlaGluSerProLysLys-Pro-OH)₂.

29. LPA selon l'une quelconque des revendications 19 à 25, dans lequel la séquence souhaitée provient de la flagelle de *Borrelia burgdorferi* ou est une séquence homologue capable de réagir avec les anticorps anti-flagelle.

30. LPA selon l'une quelconque des revendications 26 à 28, comprenant de plus une ou des séquences souhaitées provenant de la flagelle de *Borrelia burgdorferi* ou est une séquence homologue capable de réagir avec les anticorps anti-flagelle.

31. LPA selon l'une quelconque des revendications 19 à 25, dans lequel la séquence souhaitée provient de *Mycobacterium tuberculosis*.

32. LPA selon l'une quelconque des revendications 26 à 28, comprenant de plus une ou des séquences souhaitées provenant de *Mycobacterium tuberculosis*.

33. LPA selon la revendication 31 ou 32, dans lequel la séquence souhaitée comprend la protéine ESAT-6, séquence 51 à 70 ou la protéine ESAT-6, séquence 1 à 17, de *Mycobacterium tuberculosis*.

34. LPA selon l'une quelconque des revendications 31 à 33, choisi parmi :
[LPA-V] : (HO-ProLysLysProSerGluAlaValValPro-COCH₂)₂CH-NH-Lys(GlnLeuAlaAsnAsnLeuGluThrAlaThrAlaAspTrpLysGlnGlnValGly-GlnTyr-H)₂, et
[LPA-VI] : (HO-ProLysLysProSerGluAlaValValPro-COCH₂)₂N-Lys-(AlaSerAlaAlaAlaGluIleGlyAlaPheAsnTrpGlnGlnGluThrMetH)₂.

35. Composition immunologique pour susciter une réponse immune chez un animal, incluant l'homme, comprenant LPA tel que défini selon l'une quelconque des revendications 1 à 34.

36. Composition immunologique selon la revendication 35, comprenant LPA tel que défini selon l'une quelconque des revendications 1 à 34, en combinaison avec un adjuvant.

37. Composition immunologique selon la revendication 35 ou 36, qui est sous forme de vaccin.

38. Vaccin selon la revendication 37, en vue d'une administration par voie orale ou parentérale, par exemple, sous-cutanée, intramusculaire, intradermique, nasale ou pulmonaire.

39. Utilisation d'un LPA selon l'une quelconque des revendications 1 à 34, en vue de préparer une composition immunologique pour générer des anticorps chez un animal, incluant l'homme.

40. Trousse en vue d'une utilisation dans le diagnostic d'infections provoquées par des virus, des bactéries, des toxines, des allergènes, des composés apparentés au système auto-immunitaire, des composés apparentés au cancer, des molécules d'adhérence cellulaire, des facteurs neurotropes, des champignons ou des parasites, trousse qui comprend un LPA tel que défini selon l'une quelconque des revendications 1 à 34 conjointement avec un moyen pour détecter ou visualiser une liaison entre LPA et la substance à détecter.

41. Trousse en vue d'une utilisation dans le diagnostic de maladies provoquées par *Borrelia burgdorferi* au sens large, trousse qui comprend LPA tel que défini selon l'une quelconque des revendications 26 à 30 ou 32 à 34 conjointement avec un moyen pour détecter ou visualiser une liaison entre LPA et la substance à détecter.

42. Utilisation d'un LPA selon l'une quelconque des revendications 19 à 34, pour préparer une composition pharmaceutique en vue du traitement, de l'atténuation ou de la prophylaxie de maladies provoquées par des virus, des bactéries, des toxines, des allergènes, des composés apparentés au système auto-immunitaire, des composés apparentés au cancer, des molécules d'adhérence cellulaire, des fracteurs neurotropes, des champignons ou des parasites.
